# EUROPEAN PATENT SPECIFICATION

(11) **EP 4 013 740 B1**
(45) Date of publication and mention of the grant of the patent: **08.04.2026**
(21) Application number: 20851380.4
(22) Date of filing: 11.08.2020
(51) Int. Cl.: C07C 219/06, A61K 47/18, A61K 47/22, A61K 9/14, C07C 217/40, C07C 219/08, C07C 219/12, C07C 229/12, C07C 237/08, C07D 317/28, C07D 493/08, C12N 15/113, C12N 15/87

(54) **LIPIDS FOR DELIVERY OF CHARGED MATERIAL, FORMULATIONS THEREOF AND METHOD FOR MAKING SAME**
LIPIDE ZUR ABGABE VON GELADENEM MATERIAL, FORMULIERUNGEN DAVON UND VERFAHREN ZU IHRER HERSTELLUNG
LIPIDES POUR L'ADMINISTRATION D'UN MATÉRIAU CHARGÉ, LEURS FORMULATIONS ET LEUR PROCÉDÉ DE FABRICATION

(30) Priority: 12.08.2019 US 201962885677 P
(43) Date of publication of application: 22.06.2022
(73) Proprietor: Integrated Nanotherapeutics Inc., Burnaby BC V5G 4X4 (CA); The University of British Columbia, Vancouver, British Columbia V6T 1Z3 (CA)
(72) Inventor: ZAIFMAN, Joshua, Burnaby, BC V5G 4X4 (CA); CHEN, Sam, Burnaby, BC V5G 4X4 (CA); TAM, Yuen Yi, Burnaby, BC V5G 4X4 (CA); CIUFOLINI, Marco A., Burnaby, BC V5G 4X4 (CA)
(74) Representative: Barker Brettell LLP
(86) International application number: PCT/CA2020/051098
(87) International publication number: WO 2021/026647

(56) References cited:
- WO-A1-00/59474
- WO-A1-2009/064696
- WO-A1-2009/064696
- WO-A1-2011/130674
- WO-A1-2014/039501
- WO-A1-2015/121627
- WO-A1-2017/099823
- WO-A1-2017/201076
- WO-A1-2019/246203
- WO-A1-2020/051223
- WO-A1-2020/051223
- WO-A1-99/41266
- WO-A2-2004/062593
- WO-A2-2005/007810
- WO-A2-2007/073489
- WO-A2-2015/110957
- US-A- 5 010 067
- US-A1- 2002 035 082
- US-A1- 2011 059 180
- US-A1- 2014 287 024
- US-A1- 2014 369 935
- ZHANG XIAO-XIANG ET AL: "Synthesis, characterization, and in vitro transfection activity of charge-reversal amphiphiles for DNA delivery", BIOCONJUGATE CHEMISTRY, AMERICAN CHEMICAL SOCIETY, US, vol. 22, 20 April 2011 (2011-04-20), pages 690 - 699, XP002659811, ISSN: 1043-1802, [retrieved on 20110401]
- KANG JI HEE ET AL: "Self-Assembling Lipid?Peptide Hybrid Nanoparticles of Phospholipid?Nonaarginine Conjugates for Enhanced Delivery of Nucleic Acid Therapeutics", vol. 18, no. 11, 9 October 2017 (2017-10-09), US, pages 3733 - 3741, XP055794024, ISSN: 1525-7797, Retrieved from the Internet <URL:https://pubs.acs.org/doi/pdf/10.1021/acs.biomac.7b01084> DOI: 10.1021/acs.biomac.7b01084
- ZHANG ET AL., BIOCONJ. CHEM., vol. 22, 2011, pages 690 - 699, XP002659811
- NAVARRO ET AL., DRUG DELIV. TRANSL. RES., vol. 1, 2011, pages 25 - 33, XP055539074
- KOHLI ET AL., CHEM. PHYS. LIPIDS, vol. 165, 2012, pages 252 - 259, XP028458891
- IVANOVA ET AL., ORG. BIOMOL. CHEM., vol. 11, 2013, pages 7164 - 7178, XP055794019
- STUBIGER ET AL., ANAL. CHEM., vol. 86, 2014, pages 6401 - 6409, XP055187441
- VENDITTO ET AL., CHEM. COMMUN., vol. 50, 2014, pages 9109 - 9111, XP055794021
- GAO ET AL., LANGMUIR, vol. 32, 2016, pages 1601 - 1610, XP055794022
- KANG ET AL., BIOMACROMOL., vol. 18, 2017, pages 3733 - 3741, XP055794024
- AIKAWA ET AL., ACS OMEGA, vol. 2, 2017, pages 5803 - 5812, XP055794034
- TEMPRANA ET AL., PLOS ONE, vol. 12, no. 10, 2017, pages 1 - 25, XP055794026
- MAJARIKAR ET AL., J. PHOTOPOL. SCI. TECH., vol. 32, no. 1, 2019, pages 107 - 113, XP055794029
- WANG ET AL., LANGMUIR, vol. 35, 2019, pages 1672 - 1681, XP055794030
- LIU ET AL., LANGMUIR, vol. 35, 2019, pages 11217 - 11224, XP055794032

## Description

### TECHNICAL FIELD

Provided herein are lipids and formulations of such lipids. The lipids and formulations thereof are useful for the delivery of charged material, including but not limited to nucleic acid and peptides.

### BACKGROUND

The intracellular delivery of nucleic acids or other charged molecules such as peptides is facilitated by their incorporation into a delivery system such as a lipid nanoparticle (LNP). For example, ionizable lipid is the primary lipid component responsible for the efficient encapsulation of nucleic acids during the LNP manufacturing process. Moreover, the ionizable or cationic lipid facilitates the subsequent controlled release of the nucleic acid from endosomes after uptake by endocytosis in target cells.

It has been proposed that transfection or gene-delivery activity depends on the chemical structure of the ionizable lipid, such as a cationic lipid (Semple, S.C., et al., Rational design of cationic lipids for siRNA delivery. Nat Biotechnol, 2010, 28(2): p. 172-6). In particular, the lipophilic portion should bear a non-cylindrical shape. An example is a lipid having a small ionizable headgroup and a hydrocarbon moiety that widens or flares outwardly from the head group.

Currently, there is a need for ionizable or charged lipid structures of a desired shape that can be prepared using simple or convenient methods. The ability to provide such lipids could significantly advance the clinical development of treatments reliant on the delivery of nucleic acids or other charged molecules to target cells.

International patent application WO2009/064696A1 discloses sterol-modified amphiphilic lipid compounds having two or more hydrophobic tails of which at least one is a sterol, and related methods, compositions, and uses.

The journal article by Zhang et al., "Synthesis, Characterization, and In Vitro Transfection Activity of Charge-Reversal Amphiphiles for DNA Delivery", Bioconjugate Chemistry 22 (2011), p. 690-699, discloses a series of charge-reversal lipids with varying chain lengths and end functionalities.

The journal article by Kang et al., "Self-Assembling Lipid-Peptide Hybrid Nanoparticles of Phospholipid-Nonaarginine Conjugates for Enhanced Delivery of Nucleic Acid Therapeutics", Biomacomolecules 18 (2017), p. 3733-3741, discloses a carrier system comprising an arginine-rich peptide.

The compositions and methods of the present disclosure seek to address the foregoing problem and/or to provide useful alternatives to what has previously been described in the art.

### SUMMARY

The invention is as defined in the appended claims.

Provided herein is an ionizable or cationic lipid having a hydrocarbon structure that facilitates the delivery of charged material. The charged material may be negatively charged material, such as a nucleic acid, or positively charged material. The lipids described herein can be prepared in a modular manner to provide tailored hydrocarbon structures of a variety of non-cylindrical shapes to facilitate the delivery of various negatively or positively charged material.

Disclosed herein is a lipid comprising a head group having a net charge at physiological pH, and being covalently attached via an optional linker region to a lipid moiety. The lipid moiety comprises a hydrocarbon structure having two or more linked hydrocarbon chains, optionally having cis or trans C=C, at least one of the chains being covalently attached to the ionizable head group optionally via the linker region. The hydrocarbon chains are bonded to one another at a branch point at an internal carbon of the chain attached to the head group via an optional linker region, which branch point comprises an X1 functional group described further herein having an electronegative atom. The hydrocarbon chains each have between 1 and 40 or 1 and 30 carbon atoms, wherein the hydrocarbon structure in total comprises between 10 and 150 carbon atoms.

According to one embodiment, there is provided a charged lipid, including a cationic or anionic lipid comprising: a head group having a net charge at physiological pH, and being covalently attached via an optional linker region to a lipid moiety; the lipid moiety comprising a hydrocarbon structure having two or more linked hydrocarbon chains, optionally having cis or trans C=C, at least one of said chains being covalently attached to the ionizable head group via the linker region, and wherein the hydrocarbon chains are bonded to one another at a branch point at an internal carbon of the hydrocarbon chain attached to the linker region, which branch point comprises an X1 functional group, the X1 functional group being selected from: -C(O)O-, -O-, - OC(O)N(R1)-, wherein the hydrocarbon chains each have between 1 and 30 carbon atoms, wherein the hydrocarbon structure in total comprises between 10 and 150 carbon atoms, and wherein R1 is independently selected from hydrogen, optionally substituted alkyl, alkenyl, alkynyl, aryl, cycloalkyl, cycloalkylalkyl, or heterocycle.

In one embodiment, the two or more linked hydrocarbon chains have a structure of Formula III: 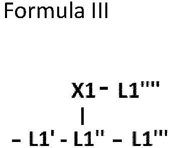 wherein L1', L1", L1‴ and L1′‴ are independently selected from hydrocarbon chains having 1 to 30 atoms, optionally comprising one or more cis or trans C=C bond and L1' is the hydrocarbon chain that is covalently attached to the linker region.

According to a further embodiment, the hydrocarbon structure further comprises a hydrophobic chain, L2, bonded to the head group via the linker region and wherein L2 is a hydrocarbon chain having 1 to 30 carbon atoms, optionally with a cis or trans C=C bond, or wherein L2 has the structure of Formula IIIa: 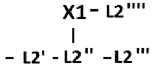 wherein L2', L2", L2‴ and L2′‴ are independently selected from hydrocarbon chains having 1 to 30 atoms, optionally comprising one or more cis or trans C=C bond, and wherein L2' is covalently attached to the linker region.

In yet a further embodiment, the hydrocarbon further comprises a hydrocarbon chain, L3, bonded to the head group via the linker region and wherein L3 has 1 to 30 carbon atoms, optionally with a cis or trans C=C bond, or has the structure of Formula IIIa: 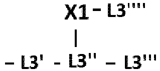 wherein L3', L3", L3‴ and L3′‴ are independently selected from hydrocarbon chains having 1 to 30 atoms, optionally comprising one or more cis or trans C=C bond, and wherein L3' is covalently attached to the linker region.

In a further embodiment, the lipid may further comprise 1 to 10 side chains S bonded to L1 via an X1 linkage, each side chain has 1 to 30 atoms, optionally with a cis or trans C=C bond, or has the structure of Formula IIIb: 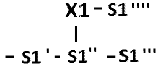 wherein S1', S1", S1‴ and S1′‴ are independently selected from hydrocarbon chains having 1 to 30 atoms, optionally comprising one or more cis or trans C=C bond and wherein S1' is bonded to a carbon of L1.

According to a further embodiment, the lipid may further comprise 1 to 10 side chains S bonded to L2 via an X1 linkage, each side chain has 1 to 30 atoms, optionally with a cis or trans C=C bond, or has the structure of Formula IIIb: 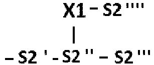 wherein S2', S2", S2‴ and S2′‴ are independently selected from hydrocarbon chains having 1 to 30 atoms, optionally comprising one or more cis or trans C=C bond, and wherein S2' is bonded to a carbon of L2.

In a further embodiment, the lipid further comprises 1 to 10 side chains S bonded to L3 via an X1 linkage, each side chain has 1 to 30 atoms, optionally with a cis or trans C=C bond, or has the structure of Formula IIIb: 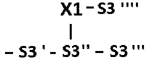 wherein S3', S3", S3‴ and S3′‴ are independently selected from hydrocarbon chains having 1 to 30 atoms, optionally comprising one or more cis or trans C=C bond, and wherein S3' is bonded to a carbon of L3.

In an alternative embodiment, one or more occurrences of X1 are biodegradable.

In yet a further embodiment, at least the hydrocarbon chain bonded to the head group via the linker region is derived from a lipid having one or more reactive groups selected from a hydroxyl, amino, and/or an amide bonded to an internal carbon atom thereof to serve as a scaffold carbon chain in the hydrocarbon structure and at least one other hydrocarbon chain in the hydrocarbon structure is derived from an acyl lipid, and wherein the X1 linkage is formed by reaction of the reactive group on the scaffold carbon chain with the carboxylic acid of the acyl chain.

In a further embodiment, the lipid is a di-hydroxy lipid.

The head group may impart to the lipid a pKₐ between 5.0 and 9.0 or a pKₐ of between 5.5 and 8.0. In an alternative embodiment, the head group has a structure of Formula I.

In a further embodiment, the linker region bonded to the hydrocarbon structure has a structure of Formula IIa or IIb.

In a further embodiment, the hydrocarbon structure is non-cylindrical in shape.

In a further embodiment, the lipid is capable of assembling into a lipid nanoparticle in combination with other lipids in aqueous solution.

In a further embodiment, the other vesicles forming lipids include phosphatidylcholine, phosphatidylglycerol, phosphatidylserine, phosphatidylethanolamine, phosphatidic acid, ceramides, sphingomyelin or a hydrophilic polymer-lipid conjugate.

Further provided is a drug delivery vehicle formulation comprising the lipid of any one of the foregoing embodiments incorporated in a lipid bilayer or monolayer thereof and comprising a nucleic acid or peptide.

In one embodiment, the nucleic acid is a small interfering RNA, a small activating RNA, a messenger RNA, a microRNA, an antisense oligonucleotide, a ribozyme, an aptamer, a plasmid, a circular DNA, a linear DNA, an antagomir, an anti-miRNA oligonucleotide or an miRNA mimic.

In a further embodiment, the drug delivery vehicle formulation comprises a lipid nanoparticle (LNP).

Further provided is a convenient method for preparing such lipids.

Such embodiment encompasses a method of preparing a hydrocarbon structure of a charged lipid for use in delivering a molecule of interest, the method comprising:
(i) providing a hydrocarbon chain having a reactive group or groups on an internal carbon thereof, the reactive group comprising an atom selected from O, P, N and/or S; and
(ii) conjugating the hydrocarbon chain to one or more acyl chains via the reactive group or groups to produce the hydrocarbon structure, and wherein the hydrocarbon structure is non-cylindrical in shape.

In one embodiment, the reactive group or groups form a respective X1 linkage upon conjugation a respective one of the acyl chains.

Further provided is a lipid produced by the foregoing method.

Further provided is a charged lipid comprising a branched lipid moiety L having the structure of Formula I:

**Formula** I: A-(V)ₘ-Z-L,

wherein
A is a head group that is charged at physiological pH;
(V)m is an optional -(CR1R2)-, and m is 1 to 10 or 2 to 6, wherein R1 and R2 are each independently: hydrogen, optionally substituted alkyl, alkenyl, alkynyl, aryl, cycloalkyl, cycloalkylalkyl, or heterocycle; and
Z-L has a structure of **Formula II, IIa** or **IIb** below:

   **Formula** II linear linker structure: X1-L_{b},

   wherein X1 is optional and X1 is selected from an ether, ester and carbamate group; and
   L_{b} is a branched lipid of **Formula IIIc;**
**Formula IIa** branched linker structure: 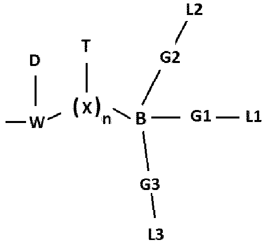
W is optional;
W, if present, is an X1 linkage, N-C(O), N-C(O)O, or N-OC(O);
wherein W is optionally substituted with D, which is an optionally substituted alkyl, alkenyl, alkynyl, aryl, cycloalkyl, cycloalkylalkyl, or heterocycle;
each occurrence of (X)ₙ is an independently selected -(CR1R2)-; n of (X)ₙ is 0 to 10; and T is optional and is an alkyl, alkenyl, alkynyl, aryl, cycloalkyl, cycloalkylalkyl, or heterocycle and wherein T is optionally substituted;
B is a carbon atom linked to L1 and L2 via respective G1 and G2;
wherein G1 and G2 are independently selected from an X1 and wherein each of G1 and G2 is independently optionally preceded and covalently bonded to B via an intervening (G)ᵤ group as B-(G)ᵤ-G1 or B-(G)ᵤ-G2, respectively, wherein (G)ᵤ is an independently selected -(CR1R2)-wherein R1 and R2 are each independently: hydrogen, optionally substituted alkyl, alkenyl, alkynyl, aryl, cycloalkyl, cycloalkylalkyl, or heterocycle and u is 0 to 16;
wherein G3 is optional and is selected from X1 and optionally covalently bonded to the B via an intervening(G)ᵤ group as B-(G)ᵤ-G3;
L1 is a branched hydrocarbon of **Formula IIIc;**
L2 is a hydrocarbon chain having 1 to 20 carbon atoms and 0 to 2 cis or trans double bonds or has the structure of **Formula IIIc;**
L3 if present is hydrogen, a linear or branched hydrocarbon chain having 1 to 20 carbon atoms and 0 to 2 cis or trans double bonds or has the structure of **Formula IIIc;**
**Formula IIb** ring structure: 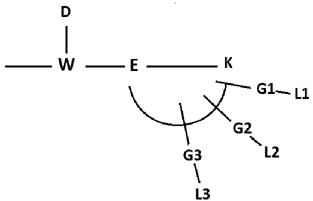
wherein the curved line represents a ring and E and K depict atoms that partially form the structure of the ring, which ring is a substituted or unsubstituted ring having 3 to 8 ring atoms;
wherein at least one of L1, L2 and L3 are bonded to a single atom in the ring, optionally via a respective G1, G2 and G3, covalently bonded to a respective one of the L1, L2 and L3 via an intervening (G)ᵤ, as G1-(G)ᵤ-L1, G2--(G)ᵤ-L2 or G3-(G)ᵤ-L3, respectively;
wherein L1 and optionally L2 and/or L3 of **Formula IIb** have the structure of **Formula IIIc:** 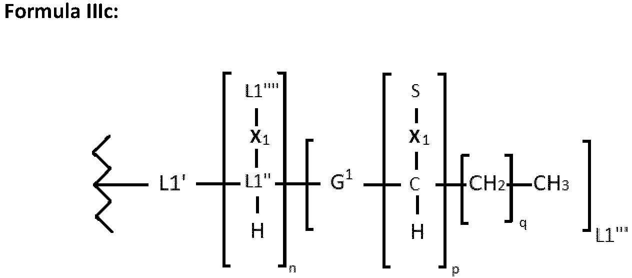
wherein an L backbone is denoted by L1' - L1"- G1- CH-[CH₂]_{q} - CH₃, and wherein the total number of carbon atoms in the L backbone is 10 to 30;
L1' is a linear hydrocarbon chain and has no heteroatoms and has 5-12, 5-10, 5-9, 6-12, 6-10, 6-9, 7-12, 7-10, or 7-9 carbon atoms and 0-3 cis or trans double bonds;
L1" is a carbon atom;
L1‴ is depicted by G1-CH-[CH₂]_{q}-CH₃ and wherein G¹ is a hydrocarbon chain of 0-4 carbon atoms, optionally having one cis or trans double bond;
wherein n is 0 to 4;
wherein p is 1 to 4;
wherein n + p is 1 to 4;
q is 0 to 20;
each X1 is independently selected from an ether, ester and carbamate group;
wherein each S and L1′‴ hydrocarbon side chain is independently:
   (a) a linear or branched terminating hydrocarbon chain with 0 to 5 cis or trans C=C and 2 to 30 carbon atoms and conjugated to one of a respective X1 at any carbon atom in its hydrocarbon chain thereof; or
   (b) a branched hydrocarbon structure of **Formula IIIc,**
wherein, optionally, the total number of L1′‴ and S hydrocarbon chains in **Formula IIIc** is 1 to 10, 1 to 9, 1 to 8, 1 to 7, 1 to 6, 1 to 5, 1 to 4 or 1 to 3;
wherein each one of the L1′‴ and S hydrocarbon chains in the lipid moiety is optionally substituted with a heteroatom, with the proviso that no more than 2 heteroatoms are substituted in the hydrocarbon chains.

In another embodiment, Z-L of **Formula I** has the structure of **Formula** II (linear linker structure):

X1-L_{b};

wherein L1' of **Formula IIIc** has 5 to 9 carbon atoms and has 0 to 2 cis or trans double bonds;
wherein G¹ of **Formula IIIc** is absent, CH₂ or CH₂CH=CH, and wherein the double bond is cis or trans;
wherein L1′‴ and S of **Formula IIIc** are independently selected from a hydrocarbon with 0-5 cis or trans CH=CH and 2 to 18 carbon atoms;
wherein a scaffold backbone of **Formula IIIc** is represented by CH₂ -L1" - G1 - CH - CH₂-CH₃ (L1‴ is 8 to 30 carbon atoms); and
wherein q is 1 to 9.

In another embodiment (V)ₘ of **Formula I** is (CH₂)₂ₘ, wherein m is 1 to 10;
Z-L has the structure of **Formula IIa** (branched linker structure): 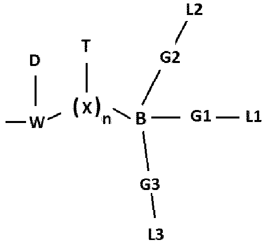
wherein W is an ether, ester or carbamate group (in any orientation) and D is absent, and (X)ₙ is (CH₂)₂ₙ, wherein n is 1 to 10;
wherein G1 and G2 are present and are preceded and covalently bonded to a respective (G)ᵤ, wherein (G)ᵤ is (CH₂)₂;
wherein G3-L3 is present and is a hydrocarbon selected from CH₃ and CH₂CH₃; or wherein G3-L3 is CH₂X1L3 and L3 is a linear or branched hydrocarbon chain having 1 to 20 carbon atoms and 0 to 2 cis or trans double bonds or has the structure of **Formula IIIc.**

In yet a further embodiment, Z-L of **Formula I** has the structure of **Formula IIb:**
**Formula IIb** ring structure: 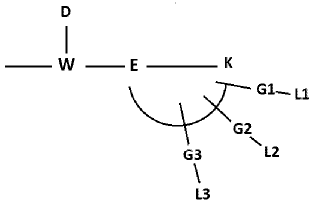 wherein the curved line represents a ring and E and K depict atoms that partially form the structure of the ring, which ring is a substituted or unsubstituted carbon ring having 3 to 6 ring atoms.

In one embodiment, the ring comprises 3 or 5 carbon atoms. In a further embodiment, at least L1 and L2 are present and are attached to the ring via respective G1 and G2 groups and wherein each G1 and G2 group is optionally preceded by a (G)ᵤ, wherein u is 0 to 10 or 0 to 6.

The head group A in **Formula I** is selected from:
(i) ionizable cationic moieties selected from the group consisting of: 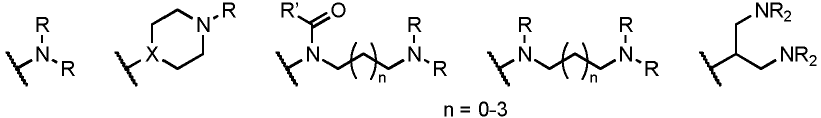 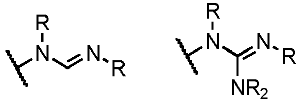
   R = H, Me, Et, Pr, Bu
   R' = C₅-C₁₇ hydrocarbon w/o *cis*/*trans*-C=C
   X = N, CH
(ii) permanently charged moieties selected from the group consisting of: 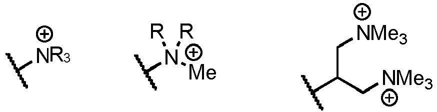 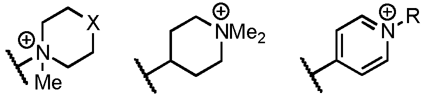 X = CH₂, NMe₂, O R = Me, Et, Pr, Bu
(iii) ionizable anionic moieties selected from the group consisting of: 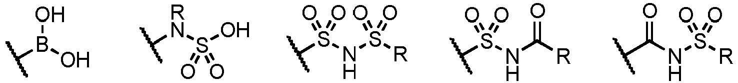 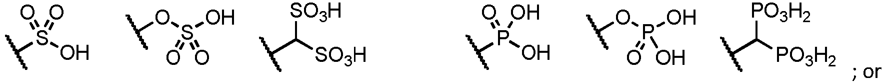
(iv) zwitterionic moieties selected from the group consisting of:

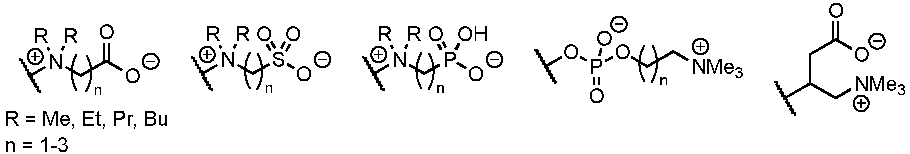

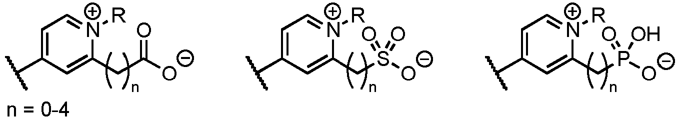

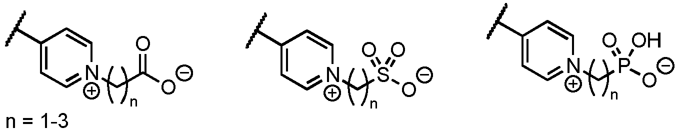

In one embodiment, the hydrocarbon structure L of **Formula I** is non-cylindrical in shape.

In a further embodiment the lipid is capable of assembling into a lipid nanoparticle in combination with other lipids in aqueous solution. In one embodiment, the other vesicles forming lipids include phosphatidylcholine, phosphatidylglycerol, phosphatidylserine, phosphatidylethanolamine, phosphatidic acid, ceramides, sphingomyelin or a hydrophilic polymer-lipid conjugate.

Further provided is a drug delivery vehicle formulation comprising the lipid described in any one of the foregoing embodiments that is incorporated in a lipid bilayer or monolayer thereof and comprising a nucleic acid, a protein or a peptide.

In one embodiment, the nucleic acid is a small interfering RNA, a small activating RNA, a messenger RNA, a microRNA, an antisense oligonucleotide, a ribozyme, an aptamer, a plasmid, a circular DNA, a linear DNA, an antagomir, an anti-miRNA oligonucleotide or an miRNA mimic.

In one embodiment, the drug delivery vehicle comprises a charged peptide.

In a further embodiment, the drug delivery vehicle is a lipid nanoparticle (LNP).

Various non-limiting embodiments are described herein.

### BRIEF DESCRIPTION OF FIGURES

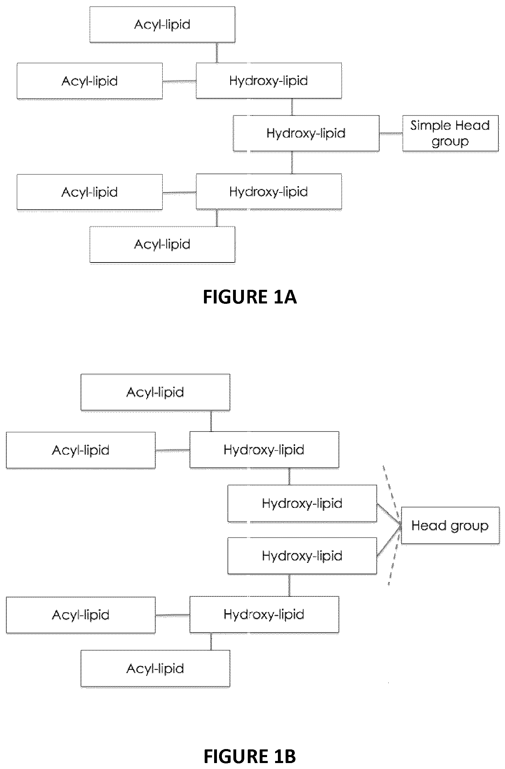

**Figure 1A** depicts amino lipids encompassed by the disclosure that have a simple head group (H) that is conjugated to one hydrocarbon chain within the lipid moiety L, optionally via a linker group. The lipid chain conjugated to the head group is derived from a hydroxy-lipid, which in turn is conjugated to one or more additional lipid chains derived from hydroxy-lipids. These lipid chains are further conjugated to one or more hydrocarbon chains derived from acyl lipids.
**Figure 1B** depicts amino lipids encompassed by the disclosure that are prepared with a head group that is conjugated to two hydrocarbons within the lipid moiety L, optionally via a linker group. The two hydrocarbons conjugated to the head group are derived from hydroxy lipids. Each of the two lipid chains derived from the hydroxy lipids is, in turn, conjugated to another respective lipid chain derived from a hydroxy-lipid and to these lipids are further conjugated one or more hydrocarbon chains derived from acyl lipids.

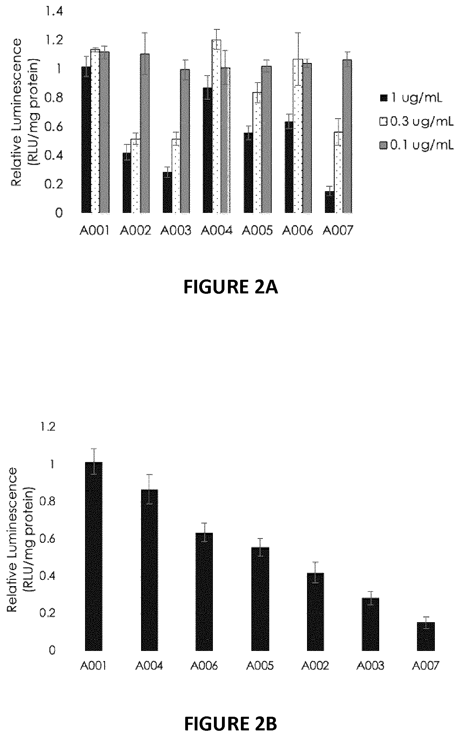

**Figure 2A** shows the activity of the amino lipids *in vitro* as evaluated in cultured luciferase expressing 22Rv1 cells. Cells were treated with lipid nanoparticle (LNP) formulations containing ionizable lipids of the disclosure and Luciferase siRNA at concentrations of 1.0 (left bar), 0.3 (middle bar) and 0.1 µg/mL (right bar). The level of luminescence was measured post treatment. The ionizable lipids incorporated in the LNPs were 2-(dimethylamino)ethyl (±)-*syn*-9,10-dilinoleoxystearate (INT-A001), 3-(diethylamino)propyl (±)-*syn*-9,10-dilinoleoxystearate (INT-A002), 3-(dimethylamino)propyl (±)-*syn*-9,10-dilinoleoxystearate (INT-A003), 2-(diethylamino)ethyl (±)-syn-9,10-dilinoleoxystearate (INT-A004), 3-(dimethylamino)propyl (12*R*)-linoleoxyoleate (INT-A005), 3-(diethylamino)propyl (*syn*-9,10,12*R*)-trilinoleoxystearate (INT-A006), and 3-(diethylamino)propyl (±)-*syn*-9,10-bis(2-hexyldecanoyloxy)stearate (INT-A007).
**Figure 2B** shows the activity of the amino lipids *in vitro* at 1 µg/mL siRNA concentration in cultured 22Rv1 cells. The level of luminescence was measured 16-24hr post treatment and the relative luminescence is ranked from highest (left) to lowest (right).

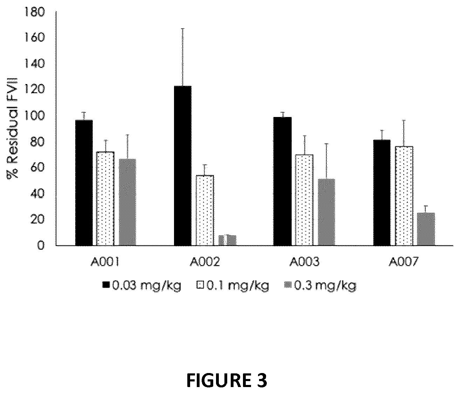

**Figure 3** shows the activity of the amino lipids *in vivo* as evaluated in a mouse FVII model. Lipid nanoparticle (LNP) formulations containing cationic lipids of the disclosure and FVII siRNA were injected via a tail vein in C57BI/6 wild-type mice and the levels of FVII in plasma was measured post injection. The mice were injected with LNP-siRNA at doses of 0.03 (left bar), 0.1 (middle bar) and 0.3 mg/kg (right bar). The cationic lipids incorporated in the LNPs were INT-A001, INT-A002, INT-A003 and INT-A007.

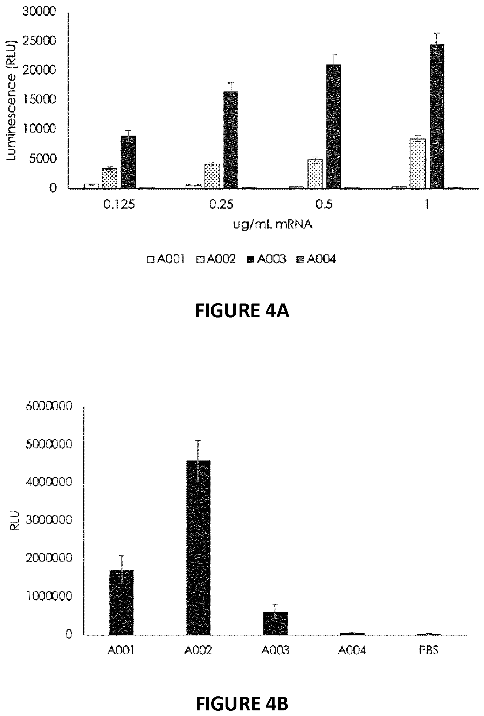

**Figure 4A** shows the activity of the ionizable lipids *in vitro* as evaluated in cultured HepG2 hepatocyte cells. Cells were treated with LNP formulations containing ionizable lipids of the disclosure and luciferase mRNA at concentrations of 0.125-1 µg/mL. The level of luminescence was measured post treatment. From left-to-right, the ionizable lipids incorporated in the LNPs were INT-A001 (white bar), INT-A002 (dotted bar), INT-A003 (black bar) and INT-A004 (gray bar).
**Figure 4B** shows the mRNA expression *in vivo* in the liver of mice. Lipid nanoparticle (LNP) formulations containing cationic lipids of the disclosure and Luciferase mRNA were injected via a tail vein in C57BI/6 wild-type mice at mRNA dose of 1 mg/kg and the luminescence in the liver was measured post injection. From left-to-right, the cationic lipids incorporated in the LNP-mRNA were INT-A001, INT-A002, INT-A003 and INT-A004. Untreated animals were injected with phosphate buffered saline (PBS).

### DETAILED DESCRIPTION

### Structure of lipid used for delivery of charged material

The lipids described herein have a head group A and a lipid moiety L having a hydrocarbon structure described below. The head group A is charged at physiological pH and in some embodiments is ionizable, although permanently charged groups are encompassed by the disclosure as well. The head group H may contain other charged groups at physiological pH, but has a net overall positive or negative charge at physiological pH. The charged lipid may be monovalent or multi-valent.

The lipid moiety L is generally composed of a hydrocarbon structure having a carbon chain or chains that function as a scaffold to conjugate additional hydrocarbon chains or groups within the hydrocarbon structure. In another advantageous embodiment, the lipid moiety L is at least partially comprised of hydrocarbon chains derived from hydroxy or other lipids with functional groups and serves as the scaffold component of the hydrocarbon structure. The hydrox-lipid(s) may, in turn, be conjugated to one or more hydrocarbon chains derived from acyl lipids.

As used herein, a "scaffold carbon chain" is a carbon chain that provides such a scaffold function by a covalent conjugation to another hydrocarbon chain in the lipid moiety L via a functional group as described herein (e.g., an "X1 functional group", "X1 linkage" or other similar convention used herein). In one example, the X1 functional group includes a group having electronegative atoms, such as N, O, S or P as an atom in the group, or optionally as a sole atom in the group, and that provides a covalent linkage of the scaffold carbon chain to one or more hydrocarbon chains, including another scaffold carbon chain. An example of a suitable X1 functional group is an ester, although other groups can be readily envisioned by those of skill in the art, including ether and carbamate groups.

The scaffold carbon chain may be derived from a precursor lipid having a hydroxyl group as described herein. Such lipids are commonly referred to as hydroxy lipids and are either naturally occurring or can be synthesized in the laboratory. In order to create an ester group, for example, the hydroxyl group of the hydrocarbon chain can be reacted with a carboxylic acid on another hydrocarbon chain via a condensation reaction. However, the method of making the hydrocarbon structure is not limited to any particular preparation method since a variety of different synthesis routes are contemplated herein.

Examples of structures of charged lipids derived from hydroxylated lipids as scaffolds are described in Figure 1A and 1B. The lipid moiety (L) may be linked to the head group A via 1, 2 or 3 hydrocarbon groups within the lipid moiety (L).

Figure 1A depicts charged lipids encompassed by the disclosure that have a simple head group (A) that is conjugated to one hydrocarbon within the lipid moiety L, optionally via a linker group. The first lipid chain in this embodiment is derived from a hydroxy-lipid. The hydroxy-lipid, in turn, may be conjugated to one or more additional lipid chains derived from hydroxy-lipids. These lipid chains are further conjugated to one or more hydrocarbon chains derived from acyl lipids.

Figure 1B depicts charged lipids encompassed by the disclosure that are prepared with a head group (A) that is conjugated to two hydrocarbons within the lipid moiety L, optionally via a linker group. In this example, the two hydrocarbons conjugated to the head group are derived from hydroxy lipids. Each of the two lipids is in turn conjugated to another respective lipid derived from a hydroxy-lipid and to these lipids are further conjugated one or more hydrocarbon chains derived from acyl lipids.

It will be understood, however, that Figure 1 is merely illustrative of select embodiments and should not be construed as limiting in any way. For example, the scaffold carbon chains alternatively could be produced from fatty acid amines, fatty acid amides and/or branched fatty acid esters.

In one example of the disclosure, the lipid comprises a head group that has a net positive or negative charge at physiological pH, and is covalently attached via a linker region (also referred to herein as a "linker") to a lipid moiety, the lipid moiety comprising a hydrocarbon structure having two or more linked hydrocarbon chains, optionally having cis or trans C=C, at least one of the chains being covalently attached to the ionizable head group via the linker region, and wherein the hydrocarbon chains are bonded to one another at a branch point at an internal carbon of the chain attached to the linker region, which branch point comprises an X1 functional group, the X1 functional group being selected from: -OC(O)-, -C(O)O-, -O-, -NR1-, -C(O)N(R1)-, N(R1)C(O)-, -OC(O)O-, -OC(O)N(R1)-, -N(R1)C(O)O-, -S-, -S-S-, -C(R1)=N-N-C(O)-, -C(O)-N-N=C(R1), -ON=C(R1)-, or -C(R1)=NO-, wherein the hydrocarbon chains each have between 1 and 30 carbon atoms, wherein the hydrocarbon structure in total comprises between 10 and 150 carbon atoms, and wherein R1 is independently selected from hydrogen, optionally substituted alkyl, alkenyl, alkynyl, aryl, cycloalkyl, cycloalkylalkyl, or heterocycle.

In a further embodiment, the hydrocarbon structure of the charged lipid has a shape that is non-cylindrical. Many phospholipids have hydrocarbon chains (formed by two fatty acid chains) linked to a glycerol backbone that form a lipophilic component that is generally cylindrical in shape. However, in certain embodiments, the hydrocarbon structure described herein forms a "flared" structure (also referred to as "frusto-conical" in shape), meaning that the hydrocarbon structure has a diameter at any point along its length that is at least 1.2, or at least 1.5, or at least 2 times greater than that of the largest diameter of the head group. Such shapes are advantageous in that they can facilitate the transfection of nucleic acids or other charged molecules to cells.

In another embodiment, the hydrocarbon structure of the charged lipid has three or more hydrocarbon chains, each of the three or more hydrocarbon chains being linked to another chain at an internal carbon thereof via the foregoing X1 linkage. In another embodiment, the lipid moiety has a hydrocarbon structure with 2 to 20 or 3 to 18 conjugated hydrocarbon chains. The hydrocarbon structure in one embodiment can be described as a lattice or matrix of connected hydrocarbon chains forming a flared, non-cylindrical structure. The connection points are optionally each biodegradable functionalities or at least 1, 2, 3, 4 or 5 of the connection points are biodegradable, as measured *in vivo* after administration to a patient.

While a variety of head groups are contemplated, in one example, the head group comprises an amino group that is ionizable, such as a terminal amine group. In one embodiment, the head group does not comprise a phosphate group. In another embodiment, the amine is a primary, secondary, tertiary or quaternary amine. If a quaternary amine is utilized in the head group, then the lipid may be non-ionizable, depending on the presence or absence of other ionizable groups in the head group. In another embodiment, the head group is non-zwitterionic.

It should be understood that each of the X1 linkages connecting the hydrocarbon chains in the hydrocarbon structure of L may be the same or differ. That is, each X1 can be independently selected from the X1 linkage groups listed above.

In one embodiment, the X1 linkage is selected from OC(O)-, -C(O)O-, and -O-. In a further alternative embodiment, the X1 linkage is any covalent bond that is biodegradable. The X1 linkage may also be pH sensitive, meaning cleavage is dependent on the pH of the surrounding solution.

In a further embodiment, the hydrocarbon structure is produced from one or more hydroxy lipids and one or more acyl lipids, wherein the one or more hydroxy lipids function as a scaffold carbon chain to conjugate said one or more acyl lipids.

In a further embodiment, the lipid has an apparent pKₐ between 5.0 and 9.0 or between 5.5 and 8.5 or between 5.0 and 8.0.

In yet a further embodiment, the charged lipid (e.g., cationic or ionizable lipid) described above can be formulated in a lipid nanoparticle, including, but not limited to, a liposome.

In a further embodiment, the charged lipid has the structure of **Formula I:**

**Formula** I: A-(V)ₘ-Z-L,

wherein
A is a head group that is charged at physiological pH;
(V)ₘ is an optional -(CR1R2)-, and m is 1 to 10 or 2 to 6, wherein R1 and R2 are each independently: hydrogen, optionally substituted alkyl, alkenyl, alkynyl, aryl, cycloalkyl, cycloalkylalkyl, or heterocycle or independently selected optionally substituted mono-, bi-, or tri-cyclic carbon ring or heteroatom ring having 4 to 12 ring atoms; and
Z-L has a structure of **Formula II, IIa** or **IIb** below.

**Formula II** is a linear linker structure: X1-L_{b},

wherein X1 is optional and X1 is selected from an ether, ester and carbamate group; and
L_{b} is a branched lipid of **Formula IIIc** below.

In another example of the disclosure, the charged lipid is an amino lipid and has the following 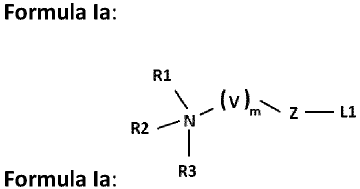
wherein R1, R2 and R3 are each independently: hydrogen, optionally substituted alkyl, alkenyl, alkynyl, aryl, cycloalkyl, cycloalkylalkyl, or heterocycle;
wherein, optionally, one of R1, R2 and R3 is absent (a lone pair), or hydrogen;
each occurrence of V is an independently selected -(CR1R2)-, and m is 1 to 10 or 2 to 6.

Z-L of **Formula I** or Z-L1 of the amino lipid of **Formula Ia** may be represented by **Formula II, Formula IIa** or **Formula IIb** depicted below:

**Formula II:** X1-L_{b},

wherein X1 is optional and X1 in one embodiment is selected from an ether, ester and carbamate group; and
L_{b} is a branched lipid of **Formula III, IIIa** or **IIIc.** 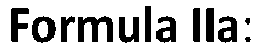 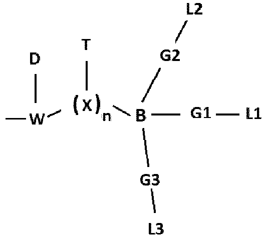
W is optional;
W, if present, is an -OC(O)-, -C(O)O-, -O-, -NR1-, -C(O)N(R1)-, N(R1)C(O)-, -NC(O)R1-,-OC(O)O-, - OC(O)N(R1)-, -N(R1)C(O)O-, -S-, -S-S-, C(R1)=N-N-C(O)-, C(O)-N-N=C(R1), -ON=C(R1), or C(R1)=NO-;
wherein W is optionally substituted with D, which is an optionally substituted alkyl, alkenyl, alkynyl, aryl, cycloalkyl, cycloalkylalkyl, or heterocycle;
each occurrence of (X)ₙ is an independently selected -(CR1R2)-, or independently selected optionally substituted mono-, bi-, or tri-cyclic carbon ring or heteroatom ring having 4 to 12 ring atoms; n of (X)ₙ is 0 to 10; and T is optional and is an optionally substituted alkyl, alkenyl, alkynyl, aryl, cycloalkyl, cycloalkylalkyl, or heterocycle;
B is a carbon, oxygen, nitrogen atom covalently attached to L1, optionally via G1; and optionally additionally attaches independently selected L2 and/or L3, optionally via respective G2 and G3 groups;
wherein G1, G2 and G3 are independently selected from -OC(O)-, -C(O)O-, -O-, -NR1-, -C(O)N(R1)-, N(R1)C(O)-, -OC(O)O-, -OC(O)N(R1)-, -N(R1)C(O)O-, -S-, -S-S-, C(R1)=N-N-C(O)-, C(O)-N-N=C(R1), -ON=C(R1), or C(R1)=NO- and wherein each of G1, G2 and G3 is independently optionally preceded and covalently bonded to a (G)ᵤ wherein G is an independently selected -(CR1R2)- and u is 0 to 10; 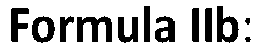 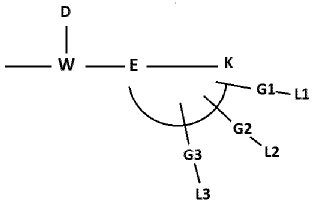
W is optional;
W, if present, is an -OC(O)-, -C(O)O-, -O-, -NR1-, -C(O)N(R1)-, N(R1)C(O)-, -NC(O)R1-,-OC(O)O-, - OC(O)N(R1)-, -N(R1)C(O)O-, -S-, -S-S-, C(R1)=N-N-C(O)-, C(O)-N-N=C(R1), -ON=C(R1), or C(R1)=NO-;
wherein W is optionally substituted with D, which is an optionally substituted alkyl, alkenyl, alkynyl, aryl, cycloalkyl, cycloalkylalkyl, or heterocycle;
wherein the curved line represents a ring and E and K depict any atoms that partially form the structure of the ring, which ring is a substituted or unsubstituted, mono-, bi-, or tri-cyclic carbon ring or mono-, bi-, or tri-cyclic heteroatom ring having 4 to 12 ring atoms;
L2 and L3 in **Formula Ia** and **Ib** are optional hydrogen atoms or hydrocarbon chains having 1 to 40 carbon atoms and optionally having one or more cis or trans C=C double bonds or have a structure of **Formula IIIa** (described hereinafter);
wherein two of L1, L2 and L3 are optionally bonded to a single atom in the ring represented by the curved line;
L, L_{b}, L1 and optionally L2 and/or L3 of the above formulas is a lipid moiety having the structure of **Formula III** described immediately below. Alternatively, the lipid moiety comprises a structure including **Formula IIIa** or **Formula IIIc** described hereinafter. 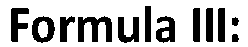 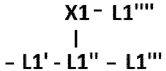
wherein L1', L1", L1‴ and L1′‴ are independently selected from hydrocarbon chains having 1 to 30 atoms, optionally comprising one or more cis or trans C=C;
wherein X1 is -OC(O)-, -C(O)O-, -O-, -NR1-, -C(O)N(R1)-, N(R1)C(O)-, -OC(O)O-, -OC(O)N(R1)-, - N(R1)C(O)O-, -S-, -S-S-, C(R1)=N-N-C(O)-, C(O)-N-N=C(R1), -ON=C(R1), or C(R1)=NO-; and
wherein, **Formula III** optionally comprises 1 to 10 side chains S, each independently selected from hydrocarbon chains having 1 to 30 atoms, optionally comprising one or more cis or trans C=C or a sterol and wherein at least one S is attached covalently to a carbon atom of L1', L1", L1‴ and/or L′‴ via a linkage that is X1.

The charged lipid (e.g., ionizable lipid) may be a mixture of enantiomers or contain a single optical isomer. As discussed above, the X1 group can be biodegradable, meaning that it can be cleaved after administration to a subject. Without being limiting, an ester bond is capable of being hydrolyzed by an esterase after administration to a patient, thereby releasing a hydrocarbon chain from the lipid. Other groups capable of being hydrolyzed by an enzyme, or in response to a pH change, are encompassed by the disclosure as well. However, it will be understood that the X1 group can be a non-biodegradable group as well.

In one embodiment, the lipid is soluble in a biocompatible alcohol, thereby facilitating its incorporation into a delivery vehicle and co-encapsulation of nucleic acid, such as a small interfering RNA, small activating RNA, messenger RNA, microRNA, antisense oligonucleotide, ribozymes, aptamer, plasmid, circular DNA, linear DNA, antagomir, anti-miRNA oligonucleotide, miRNA mimic or gene editing material.

### Head group (A)

Lipids are often conveniently represented as having a head group (denoted herein as "A") covalently attached to one or more hydrocarbon chains. Optionally, the head group A is attached to the hydrocarbon chain or chains by a linker region. Suitable head groups and linker regions are described below.

In one embodiment, the head group is selected from moieties that are ionizable, permanently charged or zwitterionic. The head group may impart either a positive or a negative charge to the lipid at a given pH value or range, including pH 7.4 (physiological pH). Examples of chemical groups that fall within each of these categories are as follows:
(i) ionizable cationic moieties: 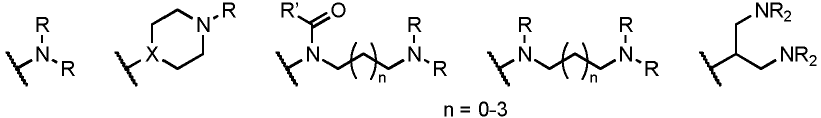 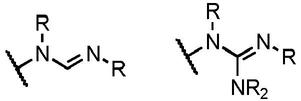
   R = H, Me, Et, Pr, Bu
   R' = C₅-C₁₇ hydrocarbon w/o *cis*/*trans*-C=C
   X = N, CH₂
(ii) permanently charged moieties: 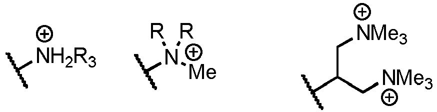 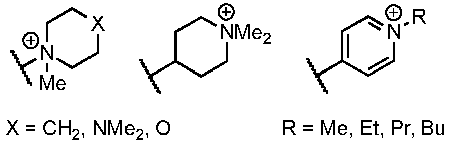
(iii) ionizable anionic moieties: 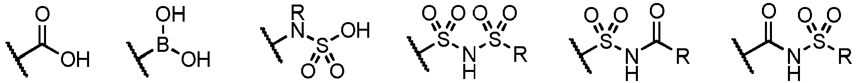 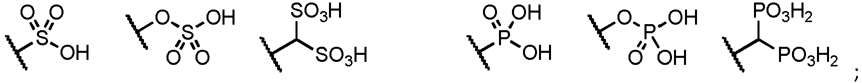 or
(iv) zwitterionic moieties:

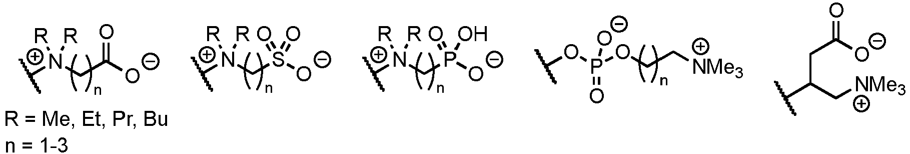

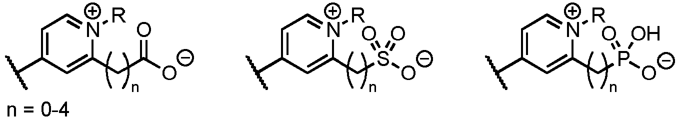

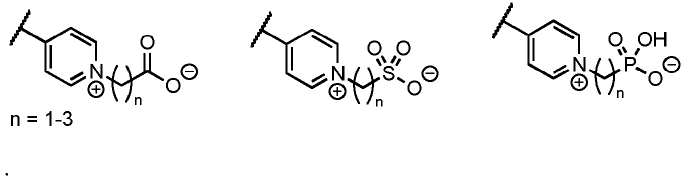

Typically, the head group A of the lipid is relatively small and charged at physiological pH. In one non-limiting embodiment, such head group comprises a terminal ionizable amine group, although although groups such as phosphate and sulfate groups are contemplated herein as well. In another embodiment, the head group has a terminal ionizable amine group that imparts an apparent pKₐ of between 5 and 8 to the ionizable lipid or a pKₐ of 5.5 to 7.5 to the ionizable lipid. It will be appreciated that the pKₐ of the amino group may be influenced by neighbouring atoms in the head group. Head groups containing sulfate or phosphate may impart an overall negative charge to the lipid.

The amine group may be a primary, secondary or tertiary amine group. The head group may contain additional amine groups, such as two or three amine groups that are the same or different.

In one embodiment, the amine portion of the head group has the following formula: 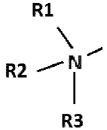 wherein R1, R2 and R3 are each independently: hydrogen, optionally substituted alkyl, alkenyl, alkynyl, aryl, cycloalkyl, cycloalkylalkyl, or heterocycle; wherein one of R1, R2 and R3 is absent (a lone pair), or hydrogen.

In those embodiments where the head group is positively charged, such group does not possess a phosphate group since this group imparts a negative charge and thus may result in a lipid that is charge neutral. However, such a group may be included in the head group provided the overall charge of the lipid is positive at physiological pH.

Optionally, the head group is conjugated to a hydrophilic polymer, including a polymer that improves circulation longevity after administration of the lipid or a formulation thereof to a patient. A non-limiting example of such a hydrophilic polymer is polyethylene glycol (PEG).

As would be appreciated by those of skill in the art, when the head group is charged, the lipid may be present as a salt. Any pharmaceutically acceptable salt is included within the scope of the disclosure.

### Optional linker region

In addition to the ionizable amine group, the lipid may comprise a region having one or more electronegative atoms that allow covalent attachment to 1, 2 or 3 lipid chains, at least one of which is a scaffold carbon chain. Such region in the lipid can be referred to as a linker region, or simply a "linker" and is optional since the hydrocarbon chain or chains may be linked directly to the head group.

The chemical structure of the linker may depend on the number of lipid chains that are attached to the head group, but may include within its structure an ester, ether, glyceride linker, a derivative of a glyceride linker, or a cyclic linker, including multi-membered rings composed of carbon or heteroatoms.

The linker region attached to the head group may have the following formula:

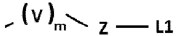

Wherein (V)ₘ-Z depicts the linker region attached at one end to the head group A and at the other end to L1, which is a hydrocarbon region.

The portion Z-L1 of the formula together may be represented by **Formula II, Formula IIa** or **Formula IIb** set forth above.

Optionally, the linker region is conjugated to a hydrophilic polymer, including a polymer that improves circulation longevity, such as polyethylene glycol (PEG).

As would be appreciated by those of skill in the art, a variety of different combinations of head-linker groups are encompassed by the present disclosure. The linker region may be characterized by the number of hydrocarbon chains attached thereto. Alternatively, the linkers may be described as linear, branched or cyclic. Examples of each category are provided below.

Non-limiting examples of head groups with linker regions that attach a single hydrocarbon chain are provided below: 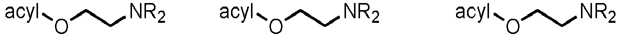 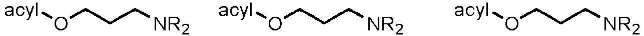 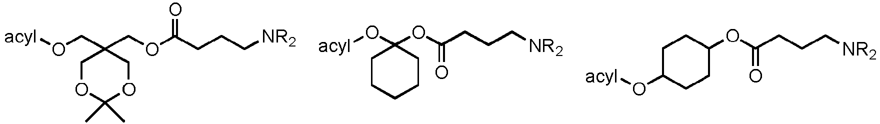 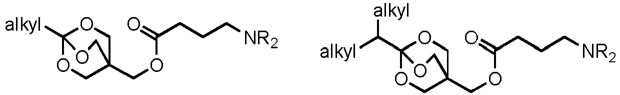 R = alkyl (C₁-C₄)

Non-limiting examples of head groups with linker regions that attach two hydrocarbon chains are provided below: 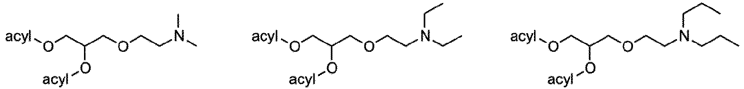 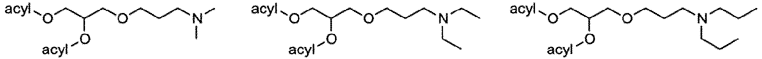 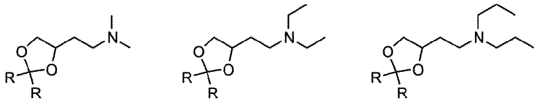 R = alkyl

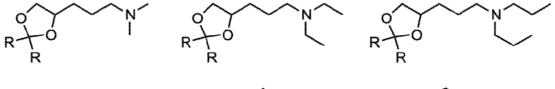

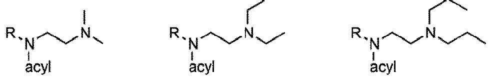

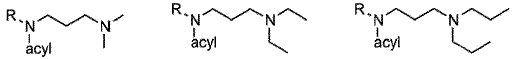

Non-limiting examples of head groups with linker regions that attach three hydrocarbon chains are provided below:

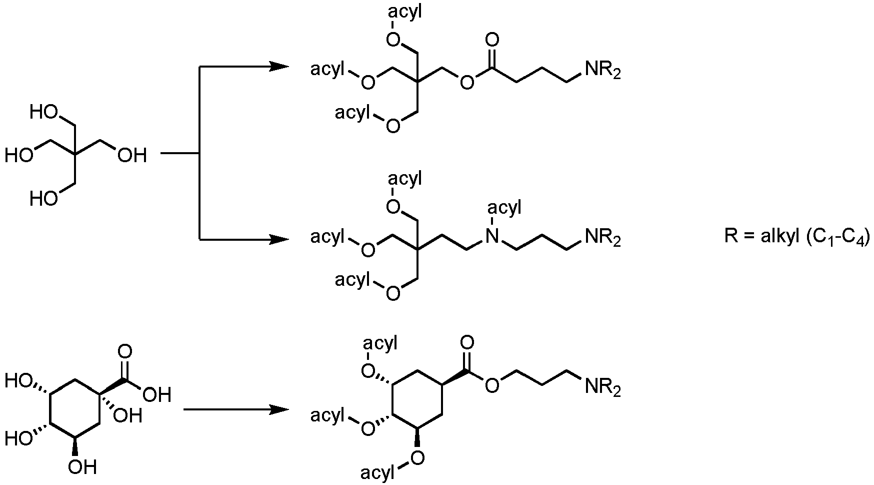

Linker regions may also be described as linear, branched or cyclic. Examples of linear, branched and cyclic linker regions are described in **Formula II, IIa** and I**Ib,** respectively, set forth above.

### Lipid Moiety L

The lipid moiety L may include 1, 2 or 3 hydrocarbon chains within the hydrocarbon structure attached to the head group directly or via the linker region. In those embodiments in which only one hydrocarbon chain is attached to the head group or linker region, additional hydrocarbon side chains, S, may be linked to an internal carbon of L1.

Thus, at a minimum, in one embodiment, the head group has attached thereto, or via a linker, an L1 that is a lipid moiety having the structure of **Formula III:** 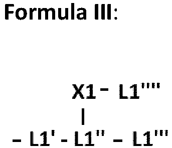
wherein L1', L1", L1‴ and L1′‴ are independently selected from hydrocarbon chains having 1 to 30 atoms, or 1 to 20 atoms, optionally comprising one or more cis or trans C=C; and
wherein X1 is -OC(O)-, -C(O)O-, -O-, -NR1-, -C(O)N(R1)-, -N(R1)C(O)-, -OC(O)O-, -OC(O)N(R1)-, - N(R1)C(O)O-, -S-, -S-S-, C(R1)=N-N-C(O)-, -C(O)-N-N=C(R1), -ON=C(R1), or -C(R1)=NO-.

Optionally, the head group has attached thereto, optionally via the linker, an L2 that is a lipid moiety, or a further L3 lipid moiety. The optional L2 and L3 lipid moieties can be each independently selected from hydrocarbon chains having 1 to 30 atoms, or 1 to 20 atoms, optionally comprising one or more cis or trans C=C, or a sterol. L2 and/or L3 optionally may be a lipid of **Formula IIIa** below. 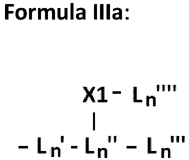 wherein Ln' is covalently attached to the linker.

According to the nomenclature of **Formula IIIa,** n is 2 if L is L2 and n is 3 if L is L3. For example, for an L2 hydrocarbon, **Formula IIIa** adopts the following nomenclature:

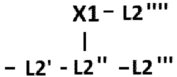

**Formula III or IIIa** optionally comprises 1 to 20 side chains S, depicted as (S)ₙ, wherein n is 0 to 20, each independently selected from hydrocarbon chains having 1 to 30 atoms, or 1 to 20 atoms, optionally comprising one or more cis or trans C=C, or a sterol and wherein (S)ₙ is/are attached covalently to a carbon atom of L1, L2 and/or L3 via a linkage that is X1 or covalently bonded to another side chain S in the hydrocarbon structure via such linkage. In one embodiment, the X1 linkage is biodegradable, such as an ester linkage. However, other linkages besides those that are biodegradable can be used in the practice of the invention.

For example, L1 may have a side chain S1' conjugated to any one of L1', L1‴ or L1′‴ via an X1 linkage. Additional side chains S1", S1‴ or S1′‴ may be attached to a carbon of L1 or any S1 side chain. In a further example, if L2 has a structure of **Formula IIIa** above, L2 may have side chain S2' attached to any one of L2', L2‴ or L2′‴. Additional side chains S2", S2‴ and S2′‴ may be attached to a carbon of L2 or any S2 side chain. Likewise, if L3 is present and has **Formula IIIa** above, it may have side chain S3' attached to any one of L3', L3‴ or L3′‴. Additional side chains S3", S3‴ and S3′‴ may be attached to a carbon of L3 or any S3 side chain.

Examples of lipids having structures encompassed by the present disclosure are depicted in Table 1 (below). Structures A-F shown in Table 1 include head groups with linkers attached to a single hydrocarbon with X1 branch points that provide scaffold attachment points to additional S hydrocarbon chains. Structures G-J shown in Table 1 include head groups with linkers attaching two hydrocarbons, L1 and L2. The lipids depicted as Structures K-M have linkers with attachment points to three hydrocarbons, L1, L2 and L3.

The annotated Structure A below exemplifies the convention used in **Formula III** herein for depicting the X1 linkages and hydrophobic regions of L1. It will be understood that each occurrence of X1 is independently selected from another X1 in the structure. As discussed, the X1 groups include any suitable functional group with electronegative atoms.

The example shown is an amino lipid having a single L1 lipid hydrocarbon chain (L1', L1" and L1‴) according to **Formula III** above with a side chain S linked to a carbon of L1:

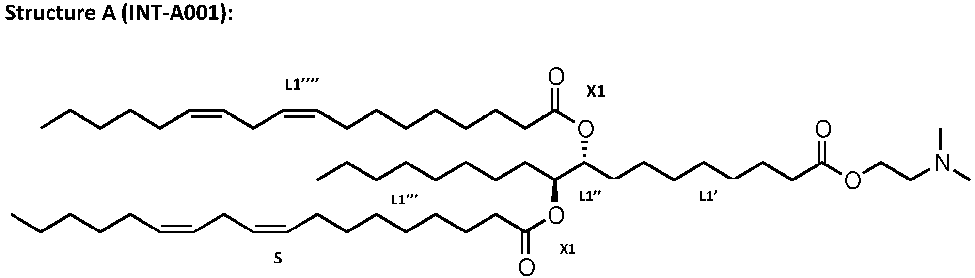

As can be seen in the Structure A (INT-A001) depicted above, L1', L1" and L1‴ together form a linear hydrocarbon backbone (referred to herein as a "scaffold carbon chain") that is derived from a di-hydroxy lipid. This hydrocarbon chain, L1'-L1"-L1‴ links L1′‴ to the L1" carbon of L1'-L1"-L1‴ via an X1 linkage and a second S "side chain" hydrocarbon chain is conjugated to L1"' via a second X1 linkage. In this structure, both X1 linkages are ester groups. However, as noted above, the X1 functional groups can be independently selected from a variety of functional groups containing O, N, P and/or S atoms.

Examples of lipids having a linker region within the head group that is attached to one, two or three hydrocarbon chains are provided in Table 1 below. For each lipid depicted, the substituents L1'/L"/L"'/L′‴ of L1 of **Formula III** are provided, as well as the optional L2 and L3 hydrocarbon chains. Also provided is the structure of the head group and linker region as defined by **Formula IIa or IIb.** In each case, the X1 group is an ester that links the hydrocarbon chains to one another to form an interconnected hydrocarbon lattice structure. As can be seen from the structures below, the interconnected hydrocarbon structures of L form a generally flared or frusto-conical shape.

**Table 1: Select amino lipids having linker region attached to one, two or three hydrocarbon chains L1, L2 and L3 forming a flared hydrocarbon structure**

| **Lipid structure** | **L1'/L"/L‴/L′‴ of Formula III:** | **Side chain S (if present) linked to another hydrocarbon via an ester (X1 =-OC(O)-) s** | **Linker and head group (terminal amine)** |
|---|---|---|---|
| | 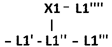 | | |
| | **X1 = -OC(O)-** | | |
| **ONE HYDROCARBON CHAIN (L1) LINKED TO HEAD GROUP** | | | |
| **A (INT-A001)** | **L1':** 7 carbon alkyl | **S:** 17 carbon chain with two | **Linker:** ester-containing |
| | **L1":** 1 carbon | | |
| 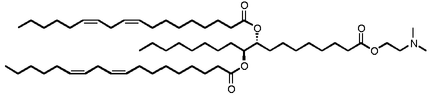 | **L1‴:** 9 carbon alkyl | cis C=C bonds linked to L1‴ | **Amine:** |
| | **L1′‴:** 17 carbon chain with two cis C=C bonds | | NMe₂ |
| **B (INT-A002)** | **L1':** 7 carbon alkyl | **S:** 17 carbon chain with two cis C=C bonds linked to L1‴ | **Linker:** ester-containing |
| 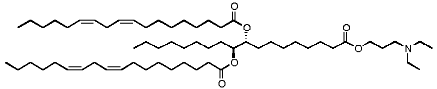 | **L1":** 1 carbon | | |
| | **L1‴:** 9 carbon alkyl | | **Amine:** |
| | **L1′‴:** 17 carbon chain with two cis C=C bonds | | NEt₂ |
| **C** | **L1':** 7 carbon alkyl | **S1':** 17 carbon chain with one cis C=C linked to L1‴ | **Linker:** ester-containing |
| 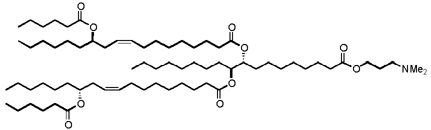 | **L1":** 1 carbon | | |
| | **L1‴:** 9 carbon alkyl | | **Amine:** |
| | **L1′‴:** 17 carbon chain with a cis C=C bond | **S1":** 5 carbon alkyl linked to S1' | NMe₂ |
| | | **S1‴:** 5 carbon alkyl linked to L1ʺʺ | |
| **D** | **L1':** 10 carbon chain with one cis C=C bond | **S1':** 17 carbon chain with one cis C=C linked to L1′‴ | **Linker:** ester containing |
| 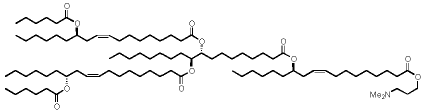 | **L1":** 1 carbon | | |
| | **L1‴:** 9 carbon alkyl | | **Amine:** |
| | **L′‴:** 19 carbon alkyl | **S1":** 5 carbonalkyl linked to S1' | NMe₂ |
| | | **S1‴:** 17 carbon chain with one cis C=C linked to L1ʺʺ | |
| | | **S1′‴:** 5 carbon alkyl linked to S1‴ | |
| **E** | **L1':** 8 carbon alkyl chain | **S1':** 5 carbon alkyl linked to L1' | **Linker:** ester containing with tri-cyclic heteratom |
| 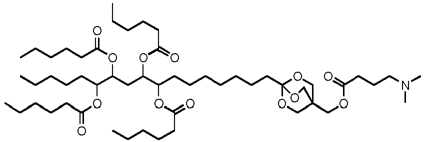 | **L1":** 1 carbon | | |
| | **L1‴:** 9 carbon alkyl | **S1':** 5 carbon alkyl linked to L1‴ | |
| | **L1′‴:** 5 carbon alkyl | | |
| | | **S1":** 5 carbon alkyl linked to L1‴ | **Amine:** |
| | | | NMe₂ |
| **F** | **L1':** 7 carbon alkyl | **S1':** 17 carbon chain with two cis C=C bonds linked to L1' via an X1 ester | **Linker:** ester containing with tri-cyclic heteroatom |
| 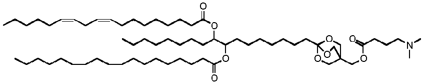 | **L1":** 1 carbon | | |
| | **L1‴:** 9 carbon alkyl | | |
| | **L1′‴:** 17 carbon chain with two cis C=C bonds linked to L1‴ via an ester | | |
| | | | **Amine:** |
| | | | NMe₂ |

| **TWO HYDROCARBON CHAINS (L1 AND L2) LINKED TO HEAD GROUP** | | | |
|---|---|---|---|
| **G** | **L1':** 11 carbon chain with one cis C=C bond | N/A | **Linker:** ester-containing |
| 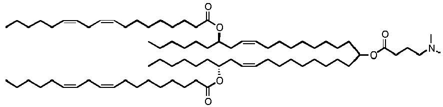 | **L1":** 1 carbon | | |
| | **L1‴:** 6 carbon alkyl | | **Amine:** |
| | **L1′‴:** 17 carbon chain with two cis C=C bonds | | NMe₂ |
| | **L2':** 11 carbon chain with one cis C=C bond | | |
| | **L2":** 1 carbon | | |
| | **L2‴:** 6 carbon alkyl | | |
| | **L2′‴:** 17 carbon chain with two cis C=C bonds | | |
| **H** | **L1':** 11 carbon chain with one cis C=C bond | **N/A** | **Linker:** |
| 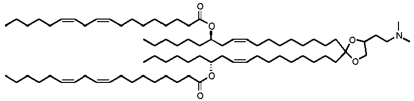 | | | Monocyclic heteroatom ring |
| | **L1":** 1 carbon | | |
| | **L1‴:** 6 carbon alkyl | | |
| | **L1′‴:** 17 carbon chain with two cis C=C bonds | | **Amine:** |
| | | | NMe₂ |
| | **L2':** 11 carbon chain with one cis C=C bond | | |
| | **L2":** 1 carbon | | |
| | **L2‴:** 6 carbon alkyl | | |
| | **L2′‴:** 17 carbon chain with two cis C=C bonds | | |
| **I** | **L1':** 8 carbon alkyl chain | **S1':** hydrocarbon of acyl chain linked to L1‴ | **Linker:** |
| 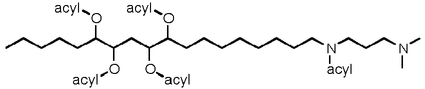 | **L1":** 1 carbon | | **Formula IIa:** |
| | **L1‴:** 9 carbon alkyl chain | | |
| | **L1′‴:** hydrocarbon of acyl chain linked via ester | **S1":** hydrocarbon of acyl chain linked to L1‴ | W-D, (X)ₙ and L3 are absent; B=N |
| | | | |
| | **L2:** carbon chain linked to N of linker via ester | | |
| | | **S1**‴**:** hydrocarbon of acyl chain linked to L1‴ | G2 = - OC(O)- |
| | | | **Amine:** |
| | | | NMe₂ |
| **J** | **L1':** 10 carbon chain with | **S1**': 17 carbon chain with cis C=C bond linked to L1"" | **Linker:** |
| 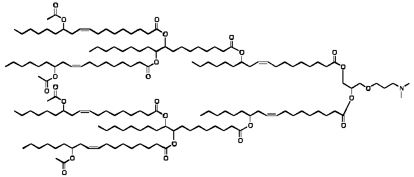 | cis C=C bond | | Glycerol |
| | **L1":** 1 carbon | | containing |
| | **L1‴:** 6 carbon alkyl | **S1':** 1 carbon chain linked to a carbon of S1' | **Formula IIa**: |
| | **L1′‴:** 17 carbon alkyl chain | | |
| | | | **W** is -O-, **D** is absent and (X)ₙ is CH₂ |
| | **L2':** 10 carbon chain with cis C=C bond | **S1":** 17 carbon chain with cis C=C bond linked to a carbon of S1' | |
| | **L2":** 1 carbon | | **B** is C |
| | **L2‴:** 6 carbon alkyl | **S1**‴ : 1 carbon chain linked to a carbon of S1" | **G1** is - CH₂OC(O)- |
| | **L2′‴:** 17 carbon alkyl chain | | **G2** is - OC(O)- |
| | | -- | **Amine:** |
| | | **S2**': 17 carbon chain with cis C=C bond | NMe₂ |
| | | **S2**": 1 carbon chain linked to a carbon of S2' | |
| | | **S2**‴: 17 carbon chain with cis C=C bond linked to a carbon of S2' | |
| | | **S2′‴:** 1 carbon chain linked to a carbon of S2‴ | |

| **THREE HYDROCARBON CHAINS (L1, L2 AND L3) LINKED TO HEAD GROUP** | | | |
|---|---|---|---|
| **K** | **L1':** 10 carbon chain with C=C cis bond | **N/A** | **Linker:** |
| 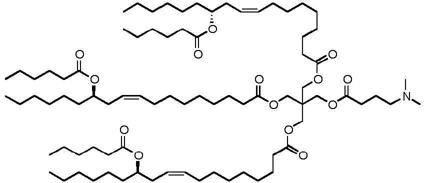 | **L1":** 1 carbon | | **Formula IIa:** |
| | **L1‴:** 6 carbon alkyl | | W = OC(O) |
| | **L1′‴:** 5 carbon alkyl | | D is absent |
| | | | Xₙ is CH₂ |
| | **L2**': 10 carbon chain with C=C cis bond | | T is absent |
| | | | B = Carbon atom |
| | **L2**": 1 carbon | | |
| | **L2‴:** 6 carbon alkyl | | G1, G2 and G3 are present and are each-OC(O)-preceded by CH₂ (Gᵤ) is 1 and R1 and R2 of (CR1R2) are each hydrogen |
| | **L2′‴:** 5 carbon alkyl | | |
| | **L3**': 10 carbon chain with C=C cis bond | | |
| | **L3**": 1 carbon | | |
| | **L3‴:** 6 carbon alkyl | | |
| | **L3′‴:** 5 carbon alkyl | | |
| | | | **Amine:** |
| | | | NMe₂ |
| **L** | **L1':** 7 carbon alkyl chain | **S1':** 5 carbon chain linked to L1' | **Linker:** |
| 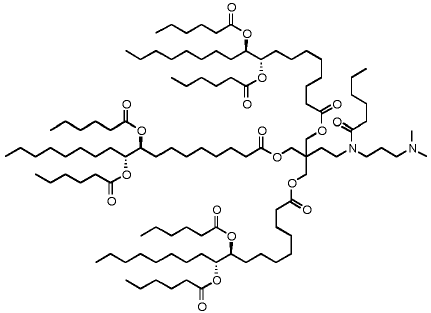 | **L1":** 1 carbon | | **Formula IIa:** |
| | **L1‴:** 8 carbon alkyl | **S2':** 5 carbon alkyl chain linked to L2‴ | |
| | **L1′‴:** 5 carbon alkyl | | **W**= - N(R1)C(O)- |
| | | **S3':** 5 carbon alkyl chain linked to L2‴ | (R1 = H) |
| | **L2':** 7 carbon alkyl chain | | **D** = 5 chain alkyl |
| | **L2":** 1 carbon | | |
| | **L2‴:** 8 carbon alkyl | | **B** = Carbon atom |
| | **L2′‴:** 5 carbon alkyl | | (**X**)ₙ is 1 and and R1 and R2 of (CR1R2) are each hydrogen |
| | **L3':** 7 carbon chain with C=C cis bond | | |
| | **L3":** 1 carbon | | |
| | **L3‴:** 8 carbon alkyl | | **G1**, **G2** and **G3** are present and are each - OC(O)-preceded by CH₂ (that is, (Gu) is 1 and R1 and R2 of (CR1R2) are each hydrogen) |
| | **L3′‴:** 5 carbon alkyl | | |
| | | | **Amine:** |
| | | | NMe₂ |
| **M** | **L1':** 10 carbon chain with C=C cis bond | **N/A** | **Linker:** |
| 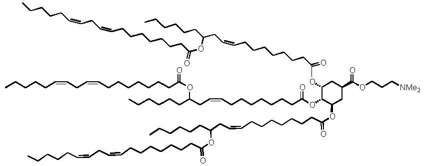 | | | Derived from quinic acid |
| | **L1":** 1 carbon | | |
| | **L1‴:** 6 carbon alkyl | | |
| | **L1′‴:** 17 carbon chain with two cis C=C bonds | | |
| | | | **Formula IIb:** |
| | **L2':** 10 carbon chain with C=C cis bond | | |
| | | | **W** =-OC(O)- |
| | **L2":** 1 carbon | | |
| | **L2‴:** 6 carbon alkyl | | **D** is not present |
| | **L2′‴:** 17 carbon chain with two cis C=C bonds | | |
| | | | (**X**)ₙ is not present (n = 0) and T is not present |
| | **L3':** 10 carbon chain with C=C cis bond | | |
| | **L3":** 1 carbon | | **E-H** = cyclohexane ring (monocyclic 6 carbon ring) |
| | **L3‴:** 6 carbon alkyl | | |
| | **L3′‴:** 17 carbon chain with two cis C=C bonds | | |
| | | | **G1, G2** and **G3** are present and are each - OC(O)- |
| | | | **Amine:** |
| | | | NMe₂ |
| **N** | **L1':** 10 carbon chain with C=C cis bond | **N/A** | **Linker:** |
| 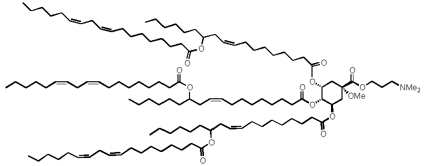 | | | Derived from quinic acid |
| | **L1":** 1 carbon | | |
| | **L1‴:** 6 carbon alkyl | | |
| | **L1′‴:** 17 carbon chain with two cis C=C bonds | | |
| | | | **Formula IIb:** |
| | **L1':** 10 carbon chain with C=C cis bond | | |
| | | | **W** =-OC(O)- |
| | **L1":** 1 carbon | | |
| | **L1‴:** 6 carbon alkyl | | **D** is not present |
| | **L1′‴:** 17 carbon chain with two cis C=C bonds | | (**X**)ₙ is not present (n = 0) and T is not present |
| | | | **Amine:** |
| | | | NMe₂ |
| | | | **E-H** = **substituted** cyclohexane ring (substituti on is methoxy, OCH₃) |
| | | | **G1, G2** and **G3** are present and are each-OC(O)- |
| **O** | **L1':** 10 carbon chain with C=C cis bond | **N**/**A** | **Linker:** |
| 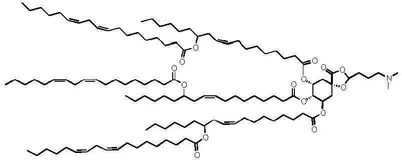 | | | **Formula IIb:** |
| | **L1":** 1 carbon | | |
| | **L1‴:** 6 carbon alkyl | | Derived from quinic acid |
| | **L1′‴:** 17 carbon chain with two cis C=C bonds | | |
| | | | |
| | **L1':** 10 carbon chain with C=C cis bond | | |
| | | | **W** =-OC(O)- |
| | **L1":** 1 carbon | | |
| | **L1‴:** 6 carbon alkyl | | **D** is not present |
| | **L1′‴:** 17 carbon chain with two cis C=C bonds | | **(X)ₙ** is not present (n = 0) and T is not present |
| | | | **E-H** = bicyclic heteroatom |
| | | | **G1, G2** and **G3** are present and are each-OC(O)- |
| | | | **Amine:** |
| | | | NMe₂ |

An alternative nomenclature for describing the lipidic moiety is **Formula** IIIc: 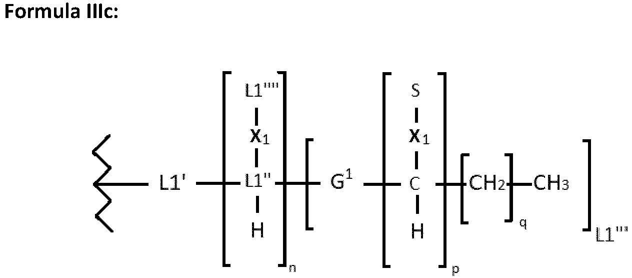
wherein an L backbone is denoted by L1' - L1"- G1- CH-[CH₂]_{q} - CH₃, and wherein the total number of carbon atoms in the L backbone is 10 to 30;
L1' is a linear hydrocarbon chain and has 2-20, 3-20, 4-20, 5-20, 6-20, 7-20, 8-20, 5-12, 5-10, 5-9, 6-12, 6-10, 6-9, 7-12, 7-10, or 7-9 carbon atoms and 0-3 cis or trans double bonds;
L1" is a carbon atom;
L1‴ is depicted by G1-CH-CH₂-CH₃;
G¹ is a hydrocarbon chain of 0-4 carbon atoms, optionally having one cis or trans double bond;
wherein n is 0 to 4;
wherein p is 1 to 4;
wherein n + p is 1 to 6 or 1 to 4;
q is 0 to 20 or 0 to 10 or 1 to 5;
each X1 is any suitable X1 group described above, or independently selected from an ether, ester and carbamate group;
wherein each S and L1′‴ hydrocarbon side chain is independently:
   (c) a linear or branched terminating hydrocarbon chain with 0 to 5 cis or trans C=C and 1 to 30 or 2 to 18 carbon atoms and conjugated to one of a respective X1 at any carbon atom in its hydrocarbon chain thereof; or
   (d) a branched structure of **Formula IIIc,**
wherein the total number of L1′‴ and S hydrocarbon chains in **Formula IIIc is** 1 to 10, 1 to 9, 1 to 8, 1 to 7, 1 to 6, 1 to 5, 1 to 4 or 1 to 3;
wherein each one of the L1′‴ and S hydrocarbon chains in the lipid moiety is optionally substituted with a heteroatom, with the proviso that no more than 4, 3 or 2 heteroatoms are substituted in the hydrocarbon chains.

The X1 ester group can be in any orientation with respect to the location of the carbonyl group as illustrated below: 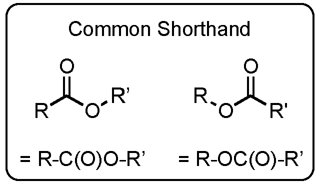
* to clarify notation used for
describing ester orientation in "G²"

In one embodiment, the total number of carbon atoms in **Formula IIIc** does not exceed 150, 125, 100, 90, 80, 70, 60 or 50.

In another embodiment, the structure of **Formula IIIc** is non-conical or flared in shape as determined from a region adjacent to the head group or linker region to a distal carbon atom on the hydrocarbon structure. As noted, such structures facilitate delivery of a cargo molecule when the lipid is formulated in a delivery vehicle.

### Scaffold carbon chain

In one embodiment, the backbone hydrocarbon chains of the L1, L2 and/or L3 lipid moieties that provide the scaffold function are derived from a fatty acid with a functional group for linkage to a side chain S. This includes fatty acids substituted with groups having atoms selected from O, N, P and/or S. Such groups facilitate the conjugation of the side chain or chains to a backbone carbon of L1, L2 and/or L3 that make up the scaffold.

For example, L1, L2 and/or L3 may be derived from a hydroxy fatty acid (HFA), which is a fatty acid having an OH group bonded at any position on its carbon chain. Without being limiting, the HFA may be a β-hydroxy fatty, an ω-hydroxy fatty acid or any (ω-1)-hydroxy fatty acid, or any other HFA with a reactive functionality at an internal carbon of the carbon backbone. The HFA may be saturated or unsaturated. Two or more hydroxy functional groups can be present on the carbon chain as well.

L1, L2 and/or L3 are alternatively derived from branched fatty acid esters of HFAs known in the art as fatty acid esters of hydroxyl fatty acids (FAHFAs). These fatty acids esters comprise a branched ester linkage between a fatty acid and an HFA. For example, 9-[(9Z)-octadecenoyloxy]octadecanoic acid is a fatty acid ester obtained by condensation of the carboxy group of oleic acid with the hydroxy group of 9-hydroxyoctadecanoic acid.

In alternative embodiments, L1, L2 and/or L3 is derived from a fatty acid amide, which may comprise ethanolamine as the amine component. Yet further, L1, L2 and/or L3 may be derived from fatty acid amines.

The scaffold carbon chain of **Formula III** may be derived from other fatty acids besides those described above. In addition, it will be appreciated that the fatty acids, in turn, can be derived from their corresponding tri-glycerides.

In **Formula III, IIIa** and **IIIb,** L1', L1" and L1‴ together form a linear hydrocarbon backbone (referred to herein as a "scaffold carbon chain"). According to **Formula IIIc** above, the scaffold carbon chain is denoted by L1' - L1"- G¹- CH-[CH₂]_{q} - CH₃, wherein the total number of carbon atoms in the scaffold is 10 to 30.

### Formulations

The cationic or ionizable lipid facilitates the encapsulation of nucleic acid, including but not limited to small interfering RNA, small activating RNA, messenger RNA, microRNA, antisense oligonucleotides, ribozymes, aptamers, plasmids, circular DNA, linear DNA, antagomir, anti-miRNA oligonucleotides and miRNA mimics, and/or gene-editing material. Alternatively or additionally, proteins and amino acids that are negatively charged can be incorporated into the delivery vehicles.

Charged lipids described herein may be used to deliver other charged molecules besides nucleic acids. This includes a wide variety of positively or negatively charged peptides, proteins, polysaccharides or carbohydrates, including both bioactive agents and prodrugs, examples of which are described below.

The cationic or ionizable lipids described herein can be administered in free form with nucleic acid or other negatively or positively charged cargo molecules, or these components can be incorporated into a delivery vehicle. Various delivery systems can be used to prepare pharmaceutical formulations. If the charged lipids and associated charged molecule are in free form, a pharmaceutically acceptable salt or excipient may be included in a pharmaceutical preparation.

The lipids of the present disclosure are particularly amenable to incorporation into nanoparticles, such as liposomes or polymer-based systems comprising lipids or other hydrophobic components, referred to herein as a "lipid nanoparticle" or "LNP".

For example, in some embodiments, the loading efficiency into a given lipid nanoparticle is 60% to 100%, 70% to 100% or most advantageously 80% to 100%.

In one embodiment, the lipids are loaded into lipid nanoparticles, such as liposomes, by mixing them with lipid formulation components, including vesicle forming lipids and optionally a sterol. As a result, lipid nanoparticles incorporating the ionizable or cationic lipids can be prepared using a wide variety of well described formulation methodologies known to those of skill in the art, including but not limited to extrusion, ethanol injection and in-line mixing. Such methods are described in Maclachlan, I. and P. Cullis, "Diffusible-PEG-lipid Stabilized Plasmid Lipid Particles", Adv. Genet., 2005. 53PA:157-188; Jeffs, L.B., et al., "A Scalable, Extrusion-free Method for Efficient Liposomal Encapsulation of Plasmid DNA", Pharm Res, 2005, 22(3):362-72; and Leung, A.K., et al., "Lipid Nanoparticles Containing siRNA Synthesized by Microfluidic Mixing Exhibit an Electron-Dense Nanostructured Core", The Journal of Physical Chemistry. C, Nanomaterials and Interfaces, 2012, 116(34): 18440-18450.

Other lipid components that may be included in the lipid nanoparticle besides the charged lipid include vesicle-forming lipids, such as phosphatidylcholine, phosphatidylglycerol, phosphatidylserine, phosphatidylethanolamine, phosphatidic acid, ceramides, or other lipids. Cholesterol may also be included in LNPs to broaden the phase transition temperature. The LNPs may also include a lipid conjugated with a hydrophilic polymer, such as, for example, distearoylphosphatidylethanolamine-PEG. Surface stabilizing functionalities such as hydrophilic polymers may be desirable to reduce clearance of the nanoparticle after administration. The LNP formulations may also include fusogenic lipids that facilitate fusion of the delivery vehicle with a target cell via endocytosis.

Suitable LNPs include, but are not limited to, liposomes prepared by extrusion by known methods or multi-lamellar vesicles (MLVs). The internal space of the liposome may comprise an entrapped agent, such as a drug, or the bilayer or bilayers may comprise such agent partitioned therein.

Another example of a suitable LNP delivery system is a stable nucleic acid-lipid particle, referred to as a SNALP. The SNALP may comprise a nucleic acid associated with the cationic lipid, a non-cationic lipid, and an optional hydrophilic polymer-lipid conjugate, such as a PEGylated lipid and a fusogenic lipid.

A lipid nanoparticle may comprise a lipophilic core. For example, the delivery vehicle can also be a nanoparticle that comprises a lipid core stabilized by a surfactant. Vesicle-forming lipids may be utilized as stabilizers. The lipid nanoparticle in another embodiment is a polymer-lipid hybrid system that comprises a polymer nanoparticle core surrounded by stabilizing lipid.

Nanoparticles may alternatively be prepared from polymers without lipids. Such nanoparticles may comprise a concentrated core of drug that is surrounded by a polymeric shell or may have a solid or a liquid dispersed throughout a polymer matrix.

The lipids described herein can also be incorporated into emulsions, which are drug delivery vehicles that contain oil droplets or an oil core. An emulsion can be lipid-stabilized. For example, an emulsion may comprise an oil filled core stabilized by an emulsifying component such as a monolayer or bilayer of lipids.

The lipids provided herein can also be formulated in micelles. Micelles are self-assembling particles composed of amphipathic lipids or polymeric components that are utilized for the delivery of agents present in the hydrophobic core.

A further class of drug delivery vehicles known to those of skill in which the charged lipid can be formulated is a carbon nanotube.

Various methods for the preparation of the foregoing delivery vehicles and the incorporation of the charged lipids therein are known and may be carried out with ease by those skilled in the art.

Certain lipids encompassed by the disclosure may form part of a carrier-free system. In such embodiments, the lipid associated with a negatively charged molecule could self-assemble into particles. An example is the formation of a lipoplex, which is an association between DNA and a cationic lipid. Such preparations can optionally include a pharmaceutically acceptable salt and/or excipient.

The delivery vehicle incorporating the cationic or ionizable lipid may also include an active agent incorporated in the vehicle, such as an anti-cancer drug or other therapeutic agent, including a pro-drug.

The delivery vehicles may optionally include lipoproteins, such as an apolipoprotein.

### Delivery of nucleic acid, genetic material, proteins, peptides or other charged agents

As discussed, the charged lipid disclosed herein facilitates the incorporation of molecules (referred to herein also as "cargo" or "cargo molecule") bearing a net negative or positive charge into a delivery vehicle and subsequent delivery to a target cell *in vitro* or *in vivo.*

In one embodiment, the molecule is genetic material, such as a nucleic acid. The nucleic acid includes, without limitation, RNA, including small interfering RNA (siRNA), small nuclear RNA (snRNA), micro RNA (miRNA), or DNA such as plasmid DNA or linear DNA. The nucleic acid length can vary and can include nucleic acid of 5-50,000 nucleotides in length. The nucleic acid can be in any form, including single stranded DNA or RNA, double stranded DNA or RNA, or hybrids thereof. Single stranded nucleic acid includes antisense oligonucleotides.

In one particularly advantageous embodiment, the cargo is an siRNA. An siRNA becomes incorporated into endogenous cellular machineries to result in mRNA breakdown, thereby preventing transcription. Since RNA is easily degraded, its incorporation into a delivery vehicle can reduce or prevent such degradation, thereby facilitating delivery to a target site.

Gene editing systems can also be incorporated into delivery vehicles comprising the charged lipid. This includes a Cas9-CRISPR, TALEN and zinc finger nuclease gene editing system. In the case of Cas9-CRISPR, a guide RNA (gRNA), together with a plasmid or mRNA encoding the Cas9 protein may be incorporated into a delivery vehicle comprising the cationic lipid described herein. Optionally, a ribonucleoprotein complex may be incorporated into a delivery vehicle comprising the cationic lipid described herein. Likewise, the disclosure includes embodiments in which genetic material encoding DNA binding and cleavage domains of a zinc finger nuclease or TALEN system are incorporated into a delivery vehicle together with the ionizable or cationic lipid.

The charged lipid can also facilitate the incorporation of proteins and peptides bearing an overall charge into a delivery vehicle. This includes both linear or non-linear peptides. Examples of peptides include bacterial/antibiotic peptides, fungal peptides, invertebrate peptides, amphibian/skin peptides, venom peptides, cancer/anticancer peptides, vaccine peptides, immune/anti-inflammatory peptides, brain peptides, endocrine peptides, ingestive peptides, gastrointestinal peptides, cardiovascular peptides, renal peptides, respiratory peptides, opiate peptides, neurotrophic peptides, and blood-brain peptides. Specific examples of peptides are provided above.

Particular examples of peptides which may associated with the charged lipids described herein are interferons and other macrophage activation factors. This includes lymphokines, muramyl dipeptide (MDP), γ-interferon, α-interferon and β-interferon, and related antiviral and tumoricidal agents; opioid peptides and neuropeptides, which includes enkaphalins, endorphins and dynorphins, and other analgesics; renin inhibitors including for example anti-hypertensive agents; cholecystokinins (CCK analogs) such as CCK, ceruletide and eledoisin, and related cardiovascular-targeting agents and CNS-targeting agents; leukotrienes and prostaglandins, including oxytocin, and other anti-inflammatory, oxytocid and abortifacient compounds; erythropoietin and deratives thereof, as well as related haematinics; LHRH analogs, such as leuprolide, buserelin and nafarelin, and related down-regulators of pituitary receptors; parathyroid hormone and other growth hormone analogs; enzymes, such as Dnase, catalase and alpha-I antitrypsin; immunosuppressants such as cyclosporin; GM-CSF and other immunomodulators; and insulin.

### Administration

In certain embodiments, the charged lipid associated with the nucleic acid or other charged molecule, which is either free or formulated in a drug delivery vehicle, is administered to treat, prevent and/or ameliorate a condition in a patient. In particular, the charged lipid in free form or formulated in a delivery vehicle together with the nucleic acid or other charged material may provide a prophylactic (preventive), ameliorative or a therapeutic benefit. A pharmaceutical composition comprising the charged lipid will be administered at any suitable dosage. In one embodiment, the lipid that is free or formulated in a drug delivery vehicle is administered parentally, i.e., intra-arterially, intravenously, subcutaneously or intramuscularly. In other embodiments, the lipid in free form or formulated in a delivery vehicle described herein may be administered topically. In still further alternative embodiments, the lipid in free form or formulated in a delivery vehicle described herein may be administered orally. In yet a further embodiment, the lipid in free form or formulated in a delivery vehicle is for pulmonary administration by aerosol or powder dispersion.

The compositions described herein may be administered to any subject, including a "patient", which as used herein includes a human or a non-human subject.

In some embodiments, the lipids described herein are used for the *in vitro* transfection of cells, including stem cells obtained from a patient and cultured cells. In one embodiment, the cells transfected are stem cells and are administered back to a patient from which they were previously obtained.

The following examples are given for the purpose of illustration only and not by way of limitation on the scope of the invention.

### EXAMPLES

### Materials and Methods

For the organic synthesis reactions described in Example 1, all reagents and solvents were purchased from commercial suppliers and used without further purification unless otherwise stated, except THF (freshly distilled from Na/benzophenone under nitrogen), and Et₃N, DMF and CH₂Cl₂ (freshly distilled from CaH₂ under nitrogen). A USP grade castor oil was purchased at a local pharmacy (Life Brand) and used as received. NMR Chemical shifts are reported in parts per million (ppm) on the δ scale and coupling constants, J, are in hertz (Hz). Spectra are referenced to the signal of the residual solvent. Multiplicities are reported as "s" (singlet), "d" (doublet), "t" (triplet), "q" (quartet), "quint" (quintet), "sept" (septet), "m" (multiplet), and further qualified as "app" (apparent) and "br" (broad).

The lipid nanoparticles (LNPs) were prepared with the neutral lipids, 1,2-distearoyl-sn-glycero-3-phosphocholine (DSPC) and 1,2-dimyristoyl-sn-glycero-3-phosphoethanolamine-N-[methoxy(polyethylene glycol)-2000] (PEG-DMPE), as well as the sterol, cholesterol. DSPC and PEG-DMPE were purchased from Avanti Polar Lipids (Alabaster, AL) and cholesterol was obtained from Sigma (St Louis, MO).

The LNPs were characterized by measuring particle size and polydispersity (Pdl). The particle size and polydispersity were determined by dynamic light scattering using a Malvern Zetasizer Nano ZS (Malvern, UK). LNP was diluted in appropriate concentrations in PBS. Number-weighted size and distribution data was used in the determination and formulations with Pdl > 0.15 were not used for further studies.

Lipid concentrations were determined by measuring total cholesterol using the Cholesterol E enzymatic assay kit from Wako Chemicals USA (Richmond, VA).

The RNA or antisense oligonucleotide encapsulation efficiency was determined using the Quant-iT Ribogreen RNA or Oligreen ssDNA Assays, respectively (Life Technologies, Burlington, ON). Briefly, LNP was incubated at 37°C for 10 min in the presence or absence of 1% Triton X-100 (Sigma-Aldrich, St. Louis, MO) followed by the addition of the Ribogreen or Oligreen reagent. The fluorescence intensity (Ex/Em: 480/520 nm) was determined and samples treated with Triton X-100 represent total nucleic acid while untreated samples represent unencapsulated nucleic acid.

Quantification of protein/peptide was performed with the use of either the BCA Protein Assay (Pierce) or the CBQCA Protein Quantitation Kit (Invitrogen) according to the manufacturer's instructions.

Apparent acid dissociation constants (pKₐ) of LNP systems were determined according to a procedure described in the literature (Jayaraman, M., et al., Maximizing the potency of siRNA lipid nanoparticles for hepatic gene silencing in vivo. Angewandte Chemie, 2012. 51(34): p. 8529-33). Briefly, 2-(p-toluidino)-6-napthalene sulfonic acid (TNS, Sigma-Aldrich, St. Louis, MO) and LNP were diluted in buffer (10 mM HEPES, 10 mM MES and 10 mM ammonium acetate) with pH ranges from 2.5 to 11. Final concentration of TNS or total lipid was 6 µM. Samples were then mixed and fluorescence intensity was measured (Ex/Em: 321/445 nm) using a Perkin Elmer LS55. A sigmoidal best fit analysis was applied and the pKₐ was measured as the pH at half-maximal fluorescence intensity.

### Example 1: Synthesis of ionizable lipids

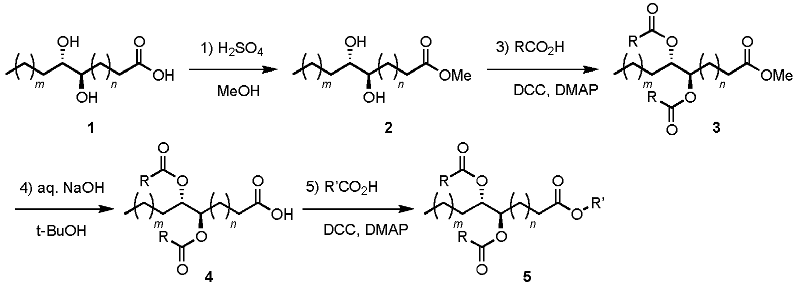

### General Procedure A - Esterification of Hydroxy Fatty Acids

The hydroxylated fatty acid (1.00 equiv.) was suspended in MeOH (0.4-0.5 M) in a round bottom flask equipped with a condenser. Concentrated sulfuric acid (0.05 equiv.) was added to the above mixture and the resultant heated at reflux, which became homogeneous in 5-15 min. After 16 h, residual MeOH was removed on a rotary evaporator and the remaining residue was partitioned between ethyl acetate (EtOAc) and saturated aqueous NaHCO₃. The aqueous layer was extracted twice with EtOAc and the combined organic layers were washed once with water, brine, dried over Na₂SO₄, gravity filtered and concentrated on a rotary evaporator to afford white powder, which was used without further purification.

### General Procedure B - Acylation of Hydroxy Fatty Acid Esters

N,N'-Dicyclohexylcarbodiimide (DCC; 1.00 equiv. per hydroxyl + additional 0.10 equiv.) was added to an ice-cold CH₂Cl₂ (0.3 M) solution of the desired carboxylic acid (1.00 equiv. per hydroxyl + additional 0.10 equiv.) in a round bottom flask under argon. Subsequently, the ice bath was removed and the resultant mixture stirred for 15 min. The reaction mixture was cooled again in an ice bath and solid hydroxy fatty acid (1.00 equiv.) was added thereto, followed by the addition of 4-dimethylaminopyridine (DMAP; 1.00 equiv. per hydroxyl + additional 0.50 equiv.). The reaction mixture was allowed to warm to room temperature over 16 h, then diluted with hexanes, stirred for 10 min and subsequently filtered through a pad of Celite^{®}. The filtrate was concentrated on a rotary evaporator to yield a crude mixture from which the desired acylated material was purified by flash column chromatography.

### General Procedure C - Saponification of Peracylated Fatty Acid Methyl Esters

Aqueous NaOH (2.0 M, 1.00 equiv.) was added to a room temperature t-BuOH (0.3 M) solution of acylated fatty acid methyl ester (1.10 equiv.) in a round bottom flask under argon. After stirring for 16 h, the reaction mixture was acidified to pH ≤2 by addition of aqueous HCI (2.0 M) and extracted three times with hexanes. The combined organic extracts were washed with brine, dried over Na₂SO₄ and concentrated on a rotary evaporator to afford the crude as a colourless oil. The crude was purified by flash column chromatography to afford the desired fatty acids.

### General Procedure D - Esterification of Peracylated Fatty Acids with Aminoalcohols

DCC (1.10 equiv.) was added to an ice-cold CH₂Cl₂ (0.2 M) solution of the fatty acid (1.00 equiv.) in a round bottom flask under argon. The ice bath was removed and the resultant stirred for 15 min. The reaction mixture was cooled again in an ice bath, neat aminoalcohol (1.20-2.00 equiv.) was added, followed by DMAP (1.20 equiv.), and the reaction mixture was allowed to warm to room temperature over 16 h. The reaction mixture was diluted with Et₂O, stirred for 10 min and subsequently filtered through a pad of Celite^{®}. The filtrate was concentrated on a rotary evaporator to yield a crude oil, which was purified by flash column chromatography to afford the desired peracylated aminolipids.

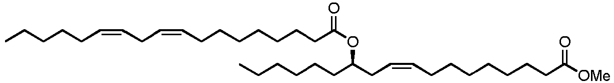

### Methyl (12R)-linoleoxyoleate

According to General Procedure B, methyl ricinoleate (500 mg, 1.60 mmol), linoleic acid (538 mg, 1.92 mmol, 1.20 equiv.), DCC (396 mg, 1.20 mmol, 2.20 equiv.) and DMAP (293 mg, 2.40 mmol, 1.50 equiv.) in CH₂Cl₂ (5 mL) afforded the title compound (875 mg, 93% yield) as a clear, colourless oil.

¹H (300 MHz, CDCl₃): 5.55-5.28 (m, 6H), 4.90 (quint, J = 6.2 Hz, 1H), 3.69 (s, 3H), 2.79 (t, J = 5.8 Hz, 2H), 2.40-2.21 (m, 6H), 2.16-1.93 (m, 6H), 1.72-1.46 (m, 8H), 1.46-1.18 (m, 32H), 1.00-0.80 (m, 6H).

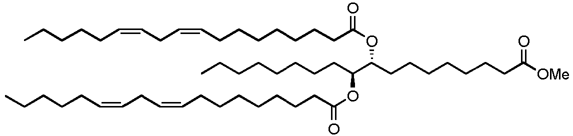

### Methyl (±)-syn-9,10-dilinoleoxystearate

According to General Procedure B, the dihydroxy stearate (1.32 g, 4.00 mmol), linoleic acid (2.47 g, 8.80 mmol, 2.20 equiv.), DCC (1.82 g, 8.80 mmol, 2.20 equiv.) and DMAP (1.22 g, 10.0 mmol, 2.50 equiv.) in CH₂Cl₂ (10 mL) afforded the title compound (2.64 g, 77% yield) as a clear, colourless oil.

¹H (300 MHz, CDCl₃): 5.47-5.28 (m, 8H), 5.04-4.94 (m, 2H), 3.68 (s, 3H), 2.79 (t, *J* = 5.8 Hz, 4H), 2.35-2.25 (m, 6H), 2.11-2.00 (m, 8H), 1.69-1.47 (m, 10H), 1.44-1.16 (m, 48 H), 0.97-0.84 (m, 9H).

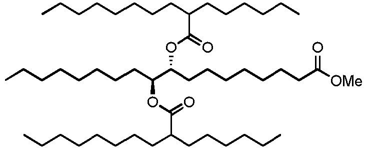

### Methyl (±)-syn-9,10-bis(2-hexyldecanoyloxy)stearate

According to General Procedure B, the dihydroxy stearate (2.31 g, 7.00 mmol), (±)-2-hexyldecanoic acid (3.77 g, 14.7 mmol, 2.10 equiv.), DCC (3.03 g, 14.7 mmol, 2.10 equiv.) and DMAP (2.14 g, 17.5 mmol, 2.50 equiv.) in CH₂Cl₂ (18 mL) afforded the title compound (4.20 g, 74% yield) as a clear, colourless oil.

¹H (300 MHz, CDCl₃): 5.06-4.96 (m, 2H), 3.68 (s, 3H), 2.31 (t, *J* = 7.6 Hz, 2H), 1.69-1.50 (m, 10H), 1.50-1.16 (m, 64H), 0.94-0.84 (m, 15H).

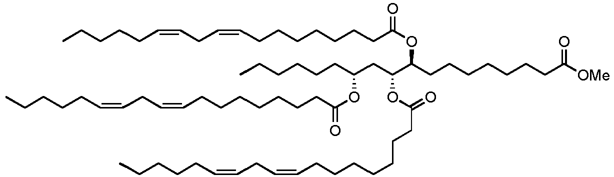

### Methyl (syn-9,10,12R)-trilinoleoxystearate

According to General Procedure B, the trihydroxy stearate (1.04 g, 3.00 mmol), linoleic acid (2.61 g, 9.30 mmol, 3.10 equiv.), DCC (1.92 g, 9.30 mmol, 3.10 equiv.) and DMAP (1.28 g, 10.5 mmol, 3.50 equiv.) in CH₂Cl₂ (10 mL) afforded the title compound (2.33 g, 68% yield) as a clear, colourless oil.

¹H (300 MHz, CDCl₃): 5.49-5.26 (m, 12H), 5.14-4.84 (m, 3H), 3.68 (s, 3H), 2.79 (br t, *J* = 6.0 Hz, 6H), 2.37-2.21 (m, 8H), 2.14-1.99 (m, 12H), 1.92-1.47 (m, 16H), 1.47-1.16 (m, 56 H), 0.96-0.84 (m, 12H).

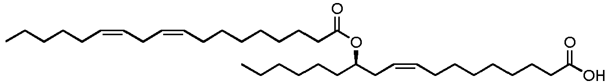

### (12R)-Linoleoxyoleic acid

According to General Procedure C, the acyl methyl ester (5.97 g, 10.4 mmol, 1.10 equiv.), aqueous NaOH (2.0 M, 4.70 mL, 1.00 equiv.) and t-BuOH (26 mL) afforded, after flash column chromatography (SiO₂, 95:5:0→90:10:0→85:12:3 hexanes/EtOAc/MeOH), the title compound (4.48 g, 85% yield) as a clear, colourless oil.

¹H (300 MHz, CDCl₃): 5.55-5.28 (m, 6H), 4.90 (quint, *J* = 6.2 Hz, 1H), 2.79 (t, *J* = 6.0 Hz, 2H), 2.43-2.21 (m, 6H), 2.14-1.96 (m, 6H), 1.73-1.47 (m, 6H), 1.46-1.18 (m, 30H), 0.99-0.81 (m, 6H).

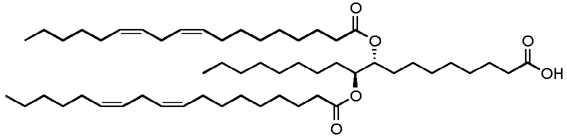

### (±)-syn-9,10-Dilinoleoxystearic acid (INT-A008)

According to General Procedure C, the diacyl methyl ester (1.88 g, 2.20 mmol, 1.10 equiv.), aqueous NaOH (2.0 M, 1.00 mL) and t-BuOH (7 mL) afforded, after flash column chromatography (SiO₂, 95:5:0→90:10:0→85:12:3 hexanes/EtOAc/MeOH), **INT-A008** (1.50 g, 89% yield) as a clear, colourless oil.

¹H (300 MHz, CDCl₃): 5.47-5.28 (m, 8H), 5.04-4.94 (m, 2H), 2.79 (br t, *J* = 6.0 Hz, 4H), 2.36 (t, *J* = 7.0 Hz, 2H), 2.30 (t, *J* = 7.5 Hz, 4H), 2.12-1.98 (m, 8H), 1.72-1.46 (m, 10H), 1.44-1.16 (m, 48 H), 0.97-0.84 (m, 9H).

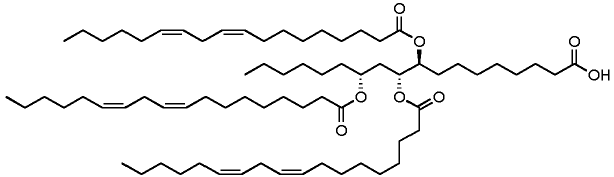

### (syn-9,10,12R)-Trilinoleoxystearic acid

According to General Procedure C, the triacyl methyl ester (2.41 g, 2.12 mmol, 1.05 equiv.), aqueous NaOH (2.0 M, 1.01 mL) and t-BuOH (7 mL) afforded, after flash column chromatography (SiO₂, 90:10:0→80:17:3 hexanes/EtOAc/MeOH), the triacylated fatty acid (1.37 g, 60% yield) as a clear, colourless oil.

¹H (300 MHz, CDCl₃): 5.47-5.26 (m, 12H), 5.14-4.84 (m, 3H), 2.79 (br t, *J* = 6.0 Hz, 6H), 2.41-2.20 (m, 8H), 2.14-1.99 (m, 12H), 1.92-1.47 (m, 16H), 1.47-1.16 (m, 56 H), 0.96-0.84 (m, 12H).

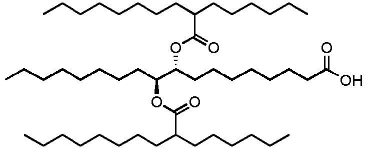

### (±)-syn-9,10-Bis(2-hexyldecanoyloxy)stearic acid (INT-A009)

According to General Procedure C, the diacyl methyl ester (4.20 g, 5.21 mmol, 1.05 equiv.), aqueous NaOH (2.0 M, 2.48 mL) and t-BuOH (7 mL) afforded, after flash column chromatography (SiO₂, 95:5:0→90:10:0→85:12:3 hexanes/EtOAc/MeOH), **INT-A009** (3.12 g, 79% yield) as a clear, colourless oil.

¹H (300 MHz, CDCl₃): 5.09-4.93 (m, 2H), 2.41-2.23 (m, 4H), 1.71-1.51 (m, 10H), 1.51-1.15 (m, 64H), 0.95-0.82 (m, 15H).

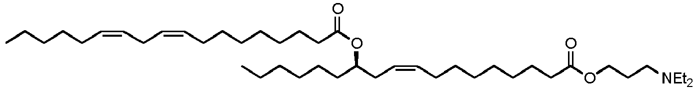

### 3-(Dimethylamino)propyl (12R)-linoleoxyoleate (INT-A005)

According to General Procedure D, the carboxylic acid (561 mg, 1.00 mmol), aminoalcohol (0.18 mL, 1.20 mmol, 1.20 equiv.), DCC (227 mg, 1.10 mmol, 1.10 equiv.) and DMAP (147 mg, 1.20 mmol, 1.20 equiv.) in CH₂Cl₂ (3.5 mL), followed by flash column chromatography (SiO₂, 88:10:2→68:30:2 hexanes/EtOAc/Et₃N), afforded **INT-A005** (639 mg, 95% yield) as a clear, colourless oil.

¹H (300 MHz, CDCl₃): 5.54-5.23 (m, 6H), 4.89 (quint, *J* = 6.0 Hz, 1H), 4.12 (t, *J* = 6.0 Hz, 2H), 2.78 (br t, *J* = 6.0 Hz, 2H), 2.58-2.45 (m, 6 H), 2.29 (br q, *J* = 6.0 Hz, 6 H), 2.12-1.96 (m, 6H), 1.83-1.72 (m, 2H), 1.69-1.47 (m, 6H), 1.44-1.18 (m, 30 H), 1.02 (t, *J* = 7.1 Hz, 6 H), 0.94-0.84 (m, 6H).

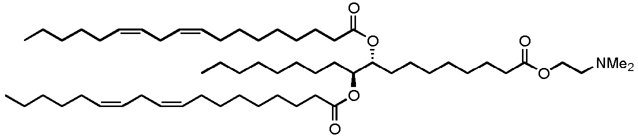

### 2-(Dimethylamino)ethyl (±)-syn-9,10-dilinoleoxystearate (INT-A001)

According to General Procedure D, the carboxylic acid (336 mg, 0.40 mmol), aminoalcohol (0.12 mL, 1.20 mmol, 3.00 equiv.), DCC (91 mg, 0.44 mmol, 1.10 equiv.) and DMAP (59 mg, 0.48 mmol, 1.20 equiv.) in CH₂Cl₂ (2 mL), followed by flash column chromatography (SiO₂, 88:20:2→68:30:2→48:50:2 hexanes/EtOAc/Et₃N), afforded **INT-A001** (255 mg, 70% yield) as a clear, colourless oil.

¹H (300 MHz, CDCl₃): 5.47-5.27 (m, 8H), 5.04-4.94 (m, 2H), 4.18 (t, *J* = 5.9 Hz, 2H), 2.79 (br t, *J* = 5.90 Hz, 4H), 2.57 (t, *J* = 5.8 Hz, 2H), 2.39-2.24 (m, 9H), 2.30 (s, 3H), 2.13-1.99 (m, 8H), 1.70-1.46 (m, 10H), 1.44-1.16 (m, 48 H), 0.97-0.84 (m, 9H).

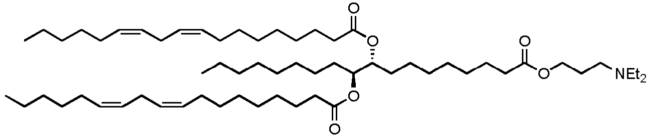

### 3-(Diethylamino)propyl (±)-syn-9,10-dilinoleoxystearate (INT-A002)

According to General Procedure D, the carboxylic acid (336 mg, 0.40 mmol), aminoalcohol (0.12 mL, 0.80 mmol, 2.00 equiv.), DCC (91 mg, 0.44 mmol, 1.10 equiv.) and DMAP (59 mg, 0.48 mmol, 1.20 equiv.) in CH₂Cl₂ (2 mL), followed by flash column chromatography (SiO₂, 78:20:2→68:30:2 hexanes/EtOAc/Et₃N), afforded **INT-A002** (272 mg, 71% yield) as a clear, colourless oil.

¹H (300 MHz, CDCl₃): 5.47-5.27 (m, 8H), 5.04-4.94 (m, 2H), 4.12 (t, *J* = 6.5 Hz, 2H), 2.79 (br t, *J* = 5.9 Hz, 4H), 2.59-2.46 (m, 6H), 2.30 (t, *J* = 7.5 Hz, 6H), 2.12-1.99 (m, 8H), 1.78 (quint, *J* = 6.9 Hz, 2H), 1.70-1.46 (m, 10H), 1.44-1.16 (m, 48 H), 1.03 (t, *J* = 7.1 Hz, 6H), 0.97-0.84 (m, 9H).

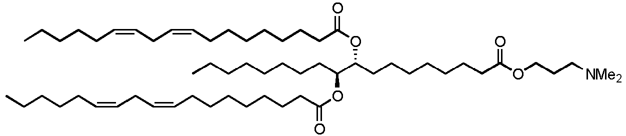

### 3-(Dimethylamino)propyl (±)-syn-9,10-dilinoleoxystearate (INT-A003)

According to General Procedure D, the carboxylic acid (421 mg, 0.50 mmol), aminoalcohol (88 µL, 0.75 mmol, 1.50 equiv.), DCC (113 mg, 0.55 mmol, 1.10 equiv.) and DMAP (73 mg, 0.60 mmol, 1.20 equiv.) in CH₂Cl₂ (3 mL), followed by flash column chromatography (SiO₂, 78:20:2→68:30:2 hexanes/EtOAc/Et₃N), afforded **INT-A003** (323 mg, 70% yield) as a clear, colourless oil.

¹H (300 MHz, CDCl₃): 5.47-5.27 (m, 8H), 5.04-4.94 (m, 2H), 4.13 (t, *J* = 6.60 Hz, 2H), 2.79 (br t, *J* = 5.90 Hz, 4H), 2.39-2.25 (m, 8H), 2.24 (s, 3H), 2.13-1.99 (m, 8H), 1.81 (quint, *J* = 6.8 Hz, 2H), 1.70-1.46 (m, 10H), 1.44-1.16 (m, 48 H), 0.97-0.84 (m, 9H).

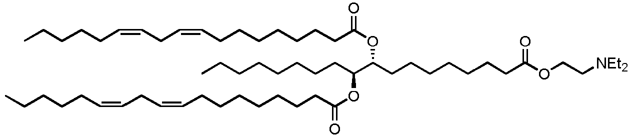

### 2-(Diethylamino)ethyl (±)-syn-9,10-dilinoleoxystearate (INT-A004)

According to General Procedure D, the carboxylic acid (421 mg, 0.50 mmol), aminoalcohol (80 µL, 0.60 mmol, 1.20 equiv.), DCC (113 mg, 0.55 mmol, 1.10 equiv.) and DMAP (73 mg, 0.60 mmol, 1.20 equiv.) in CH₂Cl₂ (3 mL), followed by flash column chromatography (SiO₂, 78:20:2→68:30:2 hexanes/EtOAc/Et₃N), afforded **INT-A004** (406 mg, 86% yield) as a clear, colourless oil.

¹H (300 MHz, CDCl₃): 5.47-5.27 (m, 8H), 5.05-4.93 (m, 2H), 4.15 (t, *J* = 6.3 Hz, 2H), 2.78 (br t, *J* = 5.9 Hz, 4H), 2.70 (t, *J* = 6.1 Hz, 2H), 2.59 (q, *J* = 7.2 Hz, 4H), 2.36-2.23 (m, 6H), 2.12-1.99 (m, 8H), 1.69-1.45 (m, 10H), 1.44-1.16 (m, 48 H), 1.05 (t, *J* = 7.1 Hz, 6H), 0.97-0.84 (m, 9H).

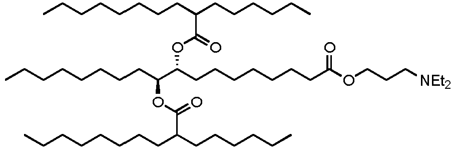

### 3-(Diethylamino)propyl (±)-syn-9,10-bis(2-hexyldecanoyloxy)stearate (INT-A007)

According to General Procedure D, the carboxylic acid (595 mg, 0.75 mmol), aminoalcohol (0.13 mL, 0.90 mmol, 1.20 equiv.), DCC (170 mg, 0.82 mmol, 1.10 equiv.) and DMAP (110 mg, 0.90 mmol, 1.20 equiv.) in CH₂Cl₂ (4 mL), followed by flash column chromatography (SiO₂, 85:15:0→80:15:5→75:20:5 hexanes/EtOAc/MeOH), afforded **INT-A007** (577 mg, 85% yield) as a clear, colourless oil.

¹H (300 MHz, CDCl₃): 5.06-4.96 (m, 2H), 4.12 (t, *J* = 6.5 Hz, 2H), 2.60-2.46 (m, 6H), 2.37-2.23 (m, 5H), 1.79 (quint, *J* = 7.2 Hz, 2H), 1.70-1.50 (m, 10H), 1.50-1.16 (m, 64H), 1.04 (t, *J* = 7.1 Hz, 6H) 0.94-0.84 (m, 15H).

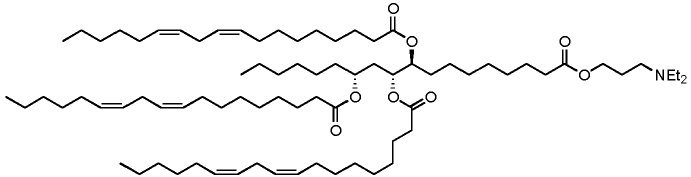

### 3-(Diethylamino)propyl (syn-9,10,12R)-trilinoleoxystearate (INT-A006)

According to General Procedure D, the carboxylic acid (560 mg, 0.50 mmol), aminoalcohol (89 µL, 0.0.60 mmol, 1.20 equiv.), DCC (113 mg, 0.55 mmol, 1.10 equiv.) and DMAP (73 mg, 0.60 mmol, 1.20 equiv.) in CH₂Cl₂ (3 mL), followed by flash column chromatography (SiO₂, 85:15:0→80:15:5→75:20:5 hexanes/EtOAc/MeOH), afforded **INT-A006** (417 mg, 68% yield) as a clear, colourless oil.

¹H (300 MHz, CDCl₃): 5.49-5.26 (m, 12H), 5.12-4.84 (m, 3H), 4.12 (t, *J* = 6.5 Hz, 2H), 2.59-2.45 (m, 6H), 2.36-2.21 (m, 8H), 2.14-1.99 (m, 12H), 1.92-1.47 (m, 18H), 1.47-1.16 (m, 56 H), 1.03 (t, *J* = 7.1 Hz, 6H), 0.96-0.84 (m, 12H).

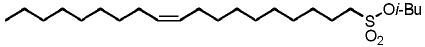

### Isobutyl (10Z)-nonadecenesulfonate

In adaptation of a procedure (M. Xie, T. S. Widlanski, Tetrahedron Lett. 1996, 37, 4443), *n*-BuLi solution (6.52 mL of 1.15 M in hexanes, 7.50 mmol, 1.50 equiv.) was added to a -78 °C 9:1 THF/DMPU (15 mL) solution of isobutyl methanesulfonate (1.22 g, 8.00 mmol, 1.60 equiv.) in a round bottom flask under argon and the resultant allowed to stir for 30 min. While still at -78 °C, a THF (2 mL) solution of oleyl iodide (1.89 g, 5.00 mmol, 1.00 equiv.) was added to the above solution and the reaction mixture was allowed to warm up over 16 h. The reaction mixture was quenched with aqueous 10% citric acid, extracted with Et₂O (2×10 mL) and the combined organics washed with water (1×10 mL), brine (1×10 mL), then dried over Na₂SO₄ and concentrated on a rotary evaporator. The crude residue was purified by flash column chromatography (98:2→95:5 hexanes/EtOAc) to afford the alkylated sulfonate (1.19 g, 46% yield) as a clear, colourless oil.

¹H (300 MHz, CDCl₃): 5.46-5.27 (m, 2H), 4.00 (d, *J* = 6.6 Hz, 2H), 3.14-3.05 (m, 2H), 2.11-1.94 (m, 5H), 1.94-1.80 (m, 2H), 1.51-1.18 (m, 26 H), 1.00 (t, *J* = 6.8 Hz, 6H), 0.90 (br t, *J* = 6.6 Hz, 3H).

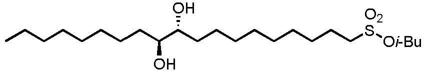

### Isobutyl (±)-syn-10,11-dihydroxynonadecanesulfonate

OsO₄ solution (0.11 mL of 4% in water, 0.02 mmol, 0.01 equiv.) was added to a room temperature 4:1 Me₂CO/H₂O (5.5 mL) solution of Isobutyl (10*Z*)-nonadecenesulfonate (709 mg, 1.76 mmol) and NMO (0.54 mL of 50% in water, 2.64 mmol, 1.50 equiv.) in a round bottom flask under argon. After stirring for 16 h, saturated aqueous NaHSO₃ was added and the reaction mixture allowed to stir for 1 h, at which point it was extracted with EtOAc (3×10 mL) and the combined organics were washed with water (1×10 mL), brine (1×10 mL), dried over Na₂SO₄ and concentrated on a rotary evaporator to afford the diol (768 mg, quantitative yield) as a white solid that was used without further purification.

¹H (300 MHz, CDCl₃): 4.00 (d, *J* = 6.6 Hz, 2H), 3.67-3.65 (m, 2H), 3.14-3.05 (m, 2H), 2.05 (sept, *J* = 6.7 Hz, 1H), 1.95-1.78 (m, 4H), 1.58-1.19 (m, 28 H), 1.00 (t, *J* = 6.8 Hz, 6H), 0.90 (br t, *J* = 6.6 Hz, 3H).

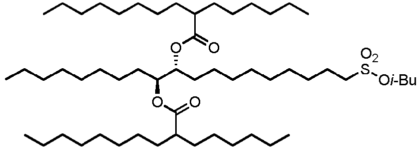

### Isobutyl (±)-syn-10,11-bis(2-hexyldecanoyloxy)nonadecanesulfonate

Solid DCC (388 mg, 1.88 mmol, 2.10 equiv.) was added to an ice-cold CH₂Cl₂ (4.5 mL) solution of (±)-2-hexyldecanoic acid (482 mg, 1.88 mmol, 2.10 equiv.) in a round bottom flask under argon. Subsequently, the ice bath was removed and the resultant mixture stirred for 15 min. The reaction mixture was cooled again in an ice bath and solid dihydroxy sulfonate (391 mg, 0.90 mmol, 1.00 equiv.) was added thereto, followed by DMAP (273 mg, 2.23 mmol, 2.50 equiv.). The reaction mixture was allowed to warm to room temperature over 16 h, then diluted with Et₂O, stirred for 10 min and subsequently filtered through a pad of Celite^{®}. The filtrate was concentrated on a rotary evaporator, then purified by flash column chromatography (SiO₂, 95:5→90:10 hexanes/EtOAc) to afford the diacylated sulfonate (574 mg, 70% yield) as a clear, colourless oil.

¹H (300 MHz, CDCl₃): 5.08-4.93 (m, 2H), 4.00 (d, *J* = 6.6 Hz, 2H), 3.67-3.65 (m, 2H), 3.14-3.05 (m, 2H), 2.38-2.24 (m, 2H), 2.05 (sept, *J* = 6.7 Hz, 1H), 1.95-1.80 (m, 2H), 1.70-1.15 (m, 74H), 1.00 (t, *J* = 6.8 Hz, 6H), 0.96-0.83 (m, 15H).

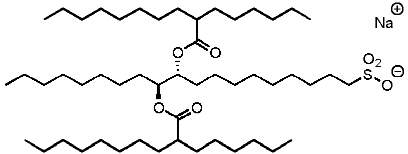

### Sodium (±)-syn-10,11-bis(2-hexyldecanoyloxy)nonadecanesulfonate (INT-A012)

Nal (82 mg, 0.55 mmol, 2.00 equiv.) was added to a room temperature Me₂CO (0.9 mL) solution of diacylated isobutyl sulfonate (250 mg, 0.27 mmol) in a sealed glass tube with a Teflon^{®} screw cap and under argon. The reaction mixture was then heated at reflux for 24 h, at which point it was diluted with Me₂CO (3 mL), cooled on ice for 2-3 h and the white precipitate was collected and washed with ice-cold Me₂CO to afford **INT-A012** as a white powder (200 mg, 84% yield)

¹H (300 MHz, CDCl₃): 5.10-4.93 (m, 2H), 2.90 (app br s, 2H), 2.46-2.22 (m, 4H), 1.70-1.15 (m, 76H), 0.96-0.83 (m, 15H).

### Example 2: Formulation of lipid nanoparticles containing nucleic acids into lipid nanoparticles (LNPs)

The lipids, INT-A001, INT-A002, INT-A003, INT-A004, INT-A005, INT-A006 and INT-A007, synthesized as described in Example 1 were formulated in lipid nanoparticles together with a nucleic acid. The nucleic acid for incorporation of the LNP as an example cargo was siRNA against Factor VII, which is a protein involved in blood coagulation. Factor VII levels can be easily measured in blood plasma by a chromogenic assay and thus represents a convenient model for determining siRNA-mediated downregulation of this factor.

Physiochemical parameters, including apparent pKₐ, particle diameter, polydispersity (PDI) and encapsulation efficiency of the nanoparticles with incorporated cationic lipid and siRNA against Factor VII were measured and reported below. The results below show that the LNPs were suitable for the encapsulation and delivery of the nucleic acid.

To prepare the LNPs, ionizable lipid, DSPC, cholesterol and PEG-DMPE were dissolved in ethanol. The siRNA was dissolved in pH 4.0-6.2 buffer composed of 10-50 mM acetate, succinate or citrate. In select cases, 10-50 mM 4-(2-Hydroxyethyl)piperazine-1-ethanesulfonic acid (HEPES) or 2-(N-Morpholino)ethanesulfonic acid (MES) buffer was used. LNP were prepared by rapidly mixing the lipid components in ethanol (in molar ratio of 50/10/38.5/1.5 of DSPC/chol/PEG-DMPE) with nucleic acids in aqueous buffer at a volumetric flow rate ratio of 1:3 (ethanol to aqueous, combined flow rate > 12 mL/min) at room temperature. Typically, siRNA/lipids ratios were targeted for 0.056 wt/µmol. The product was then dialyzed against 1 X phosphate-buffered saline (PBS) at pH 7.4 for 24 hours to remove residual ethanol and to raise the pH. PBS was refreshed after 4 hours.

As shown in Table 2 below, the INT-A001, INT-A002, INT-A003, INT-A004, INT-A005, INT-A006 and INT-A007 ionizable lipids facilitated the incorporation of FVII siRNA at high encapsulation efficiency and low polydispersity, both of which are desirable physiochemical properties for drug delivery systems. The apparent pKₐ values for these ionizable lipids were also measured as certain ionizable lipids with apparent pKₐ values between 6 to 7 were previously reported to be active in mediating gene silencing (Semple, S.C., et al., Rational design of cationic lipids for siRNA delivery. Nat Biotechnol, 2010, 28(2): p. 172-6 and Jayaraman, M., et al., Maximizing the potency of siRNA lipid nanoparticles for hepatic gene silencing in vivo. Angewandte Chemie, 2012, 51(34): p. 8529-33).

It was determined that INT-A001, INT-A002, INT-A003, INT-A004, INT-A005, INT-A006 and INT-A007 exhibit pKₐ values within the appropriate range of 6 to 7 (Table 2).

**Table 2: Physiochemical parameters of LNP containing ionizable lipids and siRNA**

| Ionizable Lipid ID | Apparent pKa | Particle Diameter (nm) | Polydispersity index (PDI) | Encapsulation Efficiency (%) |
|---|---|---|---|---|
| INT-A001 | 6.3 | 50 | 0.059 | 89 |
| INT-A002 | 6.8 | 52 | 0.063 | 97 |
| INT-A003 | 7.0 | 55 | 0.043 | 92 |
| INT-A004 | 5.8 | 58 | 0.024 | 61 |
| INT-A005 | 7.0 | 49 | 0.035 | 87 |
| INT-A006 | 6.7 | 64 | 0.020 | 83 |
| INT-A007 | 6.9 | 59 | 0.021 | 84 |

Cumulatively, this data demonstrates the suitability of these novel ionizable lipids for the encapsulation of nucleic acid in LNPs.

### Example 3: Determination of LNP activity in cultured 22Rv1 cells

The activity of the ionizable lipids *in vitro* was evaluated in luciferase expressing human prostate cells (22Rv1). LNPs containing siRNA against firefly luciferase were prepared as described in Example 2. Cells were treated with 0.1-1 µg/mL of LNPs containing siRNA for 16-24 hr and then lysed with Glo-Lysis Buffer (Promega). Equal amounts of Steady-Glo reagent (Promega) was added to each sample and the level of luminescence was determined using a Synergy LX plate reader (BioTek).Figure 2A shows the luminescence levels of various treatments. In general, a dose-dependent effect in silencing of the firefly luciferase gene was observed. Figure 2B shows the relative activity of formulations containing ionizable lipids A001-A007. The results show that the ionizable lipids of the disclosure can effectively deliver siRNA and induce gene-silencing in *vitro.* A007 was the most active at 1 ug/mL in this *in vitro* model of gene silencing.

### Example 4: Determination of LNP activity in the mouse factor VII model

The activity of the ionizable lipids *in vivo* was next evaluated in a mouse FVII model. The results show that the ionizable lipids of the disclosure can effectively deliver nucleic acids *in vivo.*

LNPs prepared as described in Example 2 containing siRNA against factor VII (FVII) were diluted with PBS such that injection volumes were maintained at 10 mL/kg body weight and administered (based on siRNA concentration) intravenously via tail vein in 6 to 8 weeks old female C57BI/6 mice (Charles River Laboratories, Wilmington, MA). At 24 hours post-injection, animals were euthanized and blood was collected via intracardiac sampling. Blood samples were allowed to coagulate at 4°C overnight and the serum was separated followed by centrifugation for 15 min at 12,000 rpm. The serum FVII levels were determined using the Biophen VII chromogenic assay (Aniara, Mason, OH) according to the protocol of the manufacturer.

Figure 3 shows the residual FVII levels in mice injected with INT-A001, INT-A002, INT-A003, INT-A005, or INT-A007 formulations. It was determined that these ionizable lipids were active in mediating gene-silencing. INT-A002 was the most active of the formulated lipids tested. The ED 50 for INT-002 was estimated as less than 0.1 mg/kg (ED 50 is the effective dose to achieve 50% gene-silencing).

### Example 5: Formulation of lipid nanoparticles containing mRNA into LNP

The ability of formulations containing INT-A001, INT-A002, INT-A003 and INT-A004 to deliver mRNA in vitro and in vivo was assessed. The results show that these ionizable lipids can effectively deliver mRNA.

LNPs were prepared as described in Example 2 containing Firefly Luciferase mRNA. As shown in Table 3 below, the INT-A001, INT-A002, INT-A003, and INT-A004 ionizable lipids facilitated the incorporation of Luciferase mRNA at high encapsulation efficiency and low polydispersity, both of which are desirable physiochemical properties for drug delivery systems.

**Table 3: Physiochemical parameters of LNP containing ionizable lipids and mRNA**

| Ionizable Lipid ID | Particle Diameter (nm) | Polydispersity index (PDI) | Encapsulation Efficiency (%) |
|---|---|---|---|
| INT-A001 | 43 | 0.064 | 96 |
| INT-A002 | 47 | 0.053 | 99 |
| INT-A003 | 49 | 0.050 | 99 |
| INT-A004 | 42 | 0.090 | 96 |

The activity of the ionizable lipids *in vitro* was evaluated in cultured HepG2 cells. LNPs containing Firefly Luciferase mRNA were prepared as described in Example 2. LNPs were diluted to 0.125-1 µg/mL with DMEM media containing 10% FBS and incubated with HepG2 cells for 16-24hr. Cells were then lysed with Glo-Lysis Buffer (Promega). Equal amounts of Steady-Glo reagent (Promega) was added to each sample and the level of luminescence was determined using a Synergy LX plate reader (BioTek). Figure 4A shows the luminescence levels of various treatments. INT-A003 formulation was the most active in delivering mRNA and inducing its expression in vitro.

To assess in vivo activity, LNPs containing Firefly Luciferase mRNA were prepared as described in Example 2. LNPs were diluted with PBS and administered at 1 mg/kg intravenously via tail vein in 6 to 8 weeks old female C57BI/6 mice (Charles River Laboratories, Wilmington, MA). At 4 hours post-injection, animals were euthanized and the livers were collected. Approximately 100 mg of liver was homogenized in 0.5 mL Glo Lysis Buffer (Promega). The homogenate was further diluted 1:4 with the lysis buffer and then 50 µL of diluted homogenate was added to 50 µL Steady-Glo reagent (Promega). The level of luminescence was determined using a Synergy LX plate reader (BioTek).Figure 4B shows the relative level of luminescence as a result of successful delivery of mRNA in vivo. INT-A002 formulation was the most active in delivering mRNA and inducing mRNA expression in vivo.

### Example 6: Formulation of lipid nanoparticles containing antisense oligonucleotides into LNPs

LNPs were prepared as described in Example 2 containing antisense oligonucleotide. As shown in Table 4 below, the INT-A001, INT-A002, INT-A003, INT-A004, INT-A005, INT-A006 and INT-A007 ionizable lipids facilitated the incorporation of antisense oligonucleotide at high encapsulation efficiency and low polydispersity, both of which are desirable physiochemical properties for drug delivery systems.

**Table 4: Physiochemical parameters of LNP containing ionizable lipids and antisense oligonucleotide**

| Ionizable Lipid ID | Particle Diameter (nm) | Polydispersity index (PDI) | Encapsulation Efficiency (%) |
|---|---|---|---|
| INT-A001 | 44 | 0.103 | 84 |
| INT-A002 | 49 | 0.096 | 92 |
| INT-A003 | 54 | 0.036 | 89 |
| INT-A004 | 45 | 0.065 | 88 |
| INT-A005 | 47 | 0.090 | 74 |
| INT-A006 | 55 | 0.062 | 88 |
| INT-A007 | 55 | 0.070 | 84 |

### Example 7: Formulation of lipid nanoparticles containing hyaluronic acid (HA) into LNPs

Hyaluronic acid was used as an example of anionic cargos. LNPs were prepared as described in Example 2 containing HA with a molecular weight of 8-15 kDa. As shown in Table 5 below, the INT-A002, INT-A003, INT-A005, INT-A006, and INT-A007 ionizable lipids facilitated the incorporation of HA.

**Table 5: Physiochemical parameters of LNP containing ionizable lipids and HA**

| Ionizable Lipid ID | Particle Diameter (nm) | Polydispersity index (PDI) | HA:Lipid (wt/wt) |
|---|---|---|---|
| INT-A002 | 83 | 0.027 | 0.093 |
| INT-A003 | 64 | 0.035 | 0.094 |
| INT-A005 | 71 | 0.096 | 0.114 |
| INT-A006 | 74 | 0.037 | 0.078 |
| INT-A007 | 58 | 0.065 | 0.096 |

### Example 8: Formulation of lipid nanoparticles containing acidic peptides into LNPs

Similar to nucleic acids, proteins or peptides with a net negative charge can be incoprorated into LNP using cationic ionizable lipids. LNPs were prepared as described in Example 2 containing an acidic peptide with a molecular weight of 4.2 kDa and a predicted net charge of -3. As shown in Table 6 below, the INT-A001, INT-A002, INT-A003, INT-A004, INT-A005, INT-A006, and INT-A007 ionizable lipids facilitated the incorporation of this acidic peptide.

**Table 6: Physiochemical parameters of LNP containing ionizable lipids and a 4.2 kDa acidic peptide**

| Ionizable Lipid ID | Particle Diameter (nm) | Polydispersity index (PDI) | Peptide:Lipid (wt/wt) |
|---|---|---|---|
| INT-A001 | 48 | 0.021 | 0.161 |
| INT-A002 | 62 | 0.015 | 0.157 |
| INT-A003 | 76 | 0.031 | 0.160 |
| INT-A004 | 40 | 0.034 | 0.158 |
| INT-A005 | 87 | 0.021 | 0.193 |
| INT-A006 | 58 | 0.070 | 0.132 |
| INT-A007 | 52 | 0.049 | 0.162 |

### Example 9: Formulation of lipid nanoparticles containing small acidic peptides into LNPs

LNPs were prepared as described in Example 2 containing an acidic peptide with a molecular weight of 2.0 kDa and a predicted net charge of -3. As shown in Table 7 below, INT-A003, INT-A004, INT-A006, and INT-A007 ionizable lipids facilitated the incorporation of this acidic peptide.

**Table 7: Physiochemical parameters of LNP containing ionizable lipids and a 2.0 kDa acidic peptide**

| Ionizable Lipid ID | Particle Diameter (nm) | Polydispersity index (PDI) | Peptide:Lipid (wt/wt) |
|---|---|---|---|
| INT-A003 | 142 | 0.025 | 0.074 |
| INT-A004 | 59 | 0.051 | 0.073 |
| INT-A006 | 94 | 0.054 | 0.061 |
| INT-A007 | 79 | 0.019 | 0.075 |

### Example 10: Formulation of lipid nanoparticles containing cationic cargo into LNPs

A basic peptide was used as an example of cationic cargos. LNPs were prepared as described in Example 2 containing a basic peptide with a molecular weight of 2.0 kDa and a predicted net charge of +4. As shown in Table 8 below, INT-A008 and INT-A009 ionizable lipids facilitated the incorporation of this basic peptide.

**Table 8: Physiochemical parameters of LNP containing ionizable lipids and a 2.0 kDa basic peptide**

| Ionizable Lipid ID | Particle Diameter (nm) | Polydispersity index (PDI) | Peptide:Lipid (wt/wt) |
|---|---|---|---|
| INT-A008 | 39 | 0.058 | 0.104 |
| INT-A009 | 39 | 0.057 | 0.121 |

## Claims

1. A lipid comprising a branched lipid moiety L having the structure of **Formula I** or a pharmaceutically acceptable salt thereof:
**Formula I:** A-(V)ₘ-Z-L,
wherein
A is a head group that is ionizable, permanently charged or zwitterionic;
(V)ₘ is an optional -(CR1R2)ₘ-, and m is 1 to 10 or 2 to 6, wherein R1 and R2 are each independently: hydrogen, optionally substituted alkyl, alkenyl, alkynyl, aryl, cycloalkyl, cycloalkylalkyl, or a heterocycle; and
Z-L has a structure of **Formula II, IIa** or **IIb** below,
and wherein L is a hydrocarbon structure and has a moiety of Formula IIIc below: 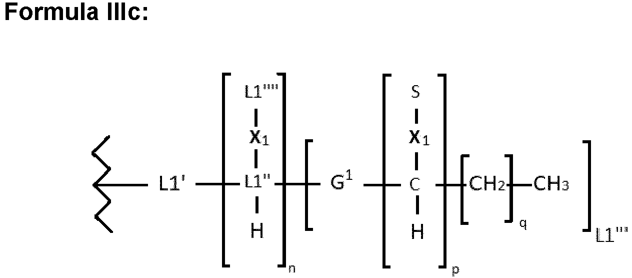
wherein a scaffold carbon chain of L is denoted by L1' - L1"- G¹- CH-[CH₂]_{q} - CH₃, and wherein the total number of carbon atoms in the L carbon backbone is 10 to 30;
L1' is a linear hydrocarbon chain having no heteroatoms and has 5-12 carbon atoms and 0-3 cis or trans double bonds;
L1" is a carbon atom;
each X1 is independently selected from an ether, ester and carbamate group;
L1‴ is a carbon backbone portion of the scaffold carbon chain L and is depicted by G¹-CH-[CH₂]_{q}-CH₃ and wherein G¹ is a hydrocarbon chain of 0-4 carbon atoms, optionally having one cis or trans double bond;
wherein n is 0 to 4;
wherein p is 1 to 4;
wherein n + p is 1 to 4;
q is 0 to 20;
wherein each S and L1ʺʺ is a hydrocarbon side chain and is independently:
(a) a linear or branched terminating hydrocarbon chain with 0 to 5 cis or trans C=C and 2 to 30 carbon atoms and conjugated to one of a respective X1 at any carbon atom in its hydrocarbon chain thereof; or
(b) a branched hydrocarbon structure of **Formula IIIc,**
wherein each one of the L1ʺʺ and S hydrocarbon chains in the lipid moiety is optionally substituted with a heteroatom, with the proviso that no more than 2 heteroatoms are substituted in the hydrocarbon chains,
**Formula II** linear linker structure: X1-L_{b},
wherein X1 is optional and X1 is selected from an ether, ester and carbamate group; and
L_{b} is a branched lipid of **Formula IIIc;** 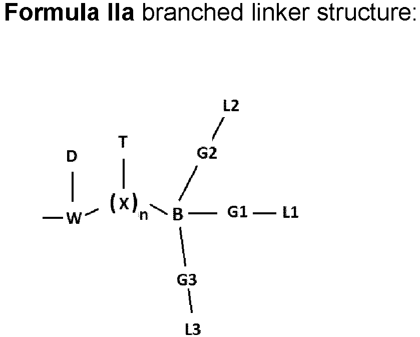
W is optional;
W, if present, is an X1 linkage, N-C(O), N-C(O)O, or N-OC(O);
wherein W is optionally substituted with D, which is an optionally substituted alkyl, alkenyl, alkynyl, aryl, cycloalkyl, cycloalkylalkyl, or heterocycle;
each occurrence of (X)ₙ is an independently selected -(CR1R2)ₙ-; n of (X)ₙ is 0 to 10; and T is optional and is an alkyl, alkenyl, alkynyl, aryl, cycloalkyl, cycloalkylalkyl, or heterocycle and wherein T is optionally substituted;
B is a carbon atom linked to L1 and L2 via respective G1 and G2;
wherein G1 and G2 are independently selected from an X1;
wherein each of G1 and G2 is independently optionally covalently bonded to B via an intervening (G)ᵤ group as B-(G)ᵤ-G1 or B-(G)ᵤ-G2, respectively;
wherein (G)ᵤ is an independently selected -(CR1R2)ᵤ- wherein R1 and R2 are each independently: hydrogen, optionally substituted alkyl, alkenyl, alkynyl, aryl, cycloalkyl, cycloalkylalkyl, or heterocycle and u is 0 to 16;
wherein G3 is optional and is selected from X1 and optionally covalently bonded to the B via an intervening(G)ᵤ group as B-(G)ᵤ-G3;
L1 is a branched hydrocarbon of **Formula IIIc;**
L2 is a hydrocarbon chain having 1 to 20 carbon atoms and 0 to 2 cis or trans double bonds or has the structure of **Formula IIIc;**
L3 if present is hydrogen, a linear or branched hydrocarbon chain having 1 to 20 carbon atoms and 0 to 2 cis or trans double bonds or has the structure of **Formula IIIc;** 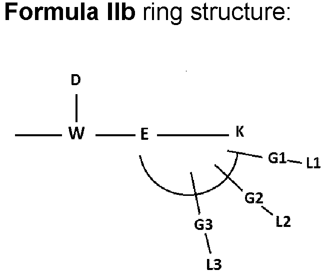
wherein the curved line represents a ring and E and K depict atoms that partially form the structure of the ring, which ring is a substituted or unsubstituted ring having 3 to 8 ring atoms;
wherein at least one of L1, L2 and L3 are bonded to a single atom in the ring, optionally via a respective G1, G2 and G3, wherein each of G1, G2 and G3 is independently optionally covalently bonded to a respective one of the L1, L2 and L3 via an intervening (G)ᵤ, as G1-(G)ᵤ-L1, G2--(G)ᵤ-L2 or G3-(G)ᵤ-L3, respectively;
wherein L1 and optionally L2 and/or L3 of **Formula IIb** have the structure of **Formula IIIc;** and
wherein the A moiety is selected from one of:
(i) ionizable cationic moieties selected from the group consisting of: 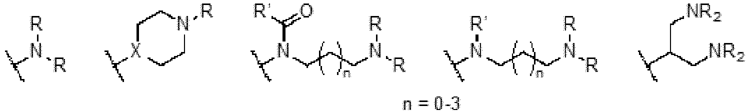 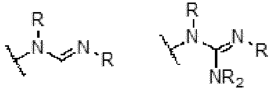
R = H, Me, Et, Pr, Bu
R' = C₅-C₁₇ hydrocarbon w/o cis/trans-C=C
X = N, CH
;
(ii) permanently charged moieties selected from the group consisting of: 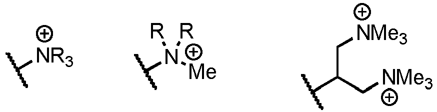 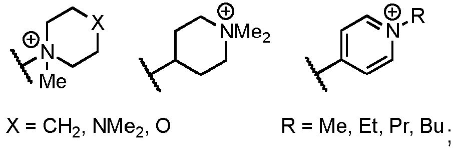
(iii) ionizable anionic moieties selected from the group consisting of: 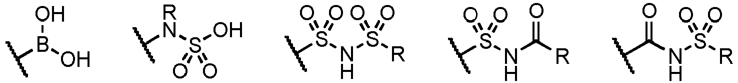 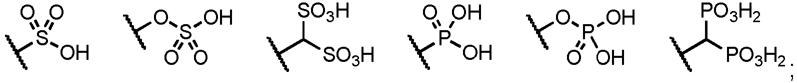 or
(iv) zwitterionic moieties selected from the group consisting of:

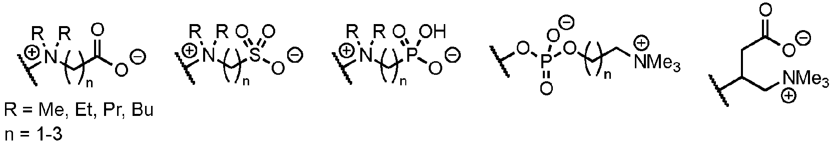

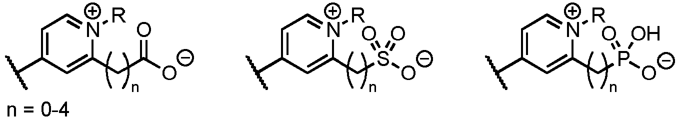

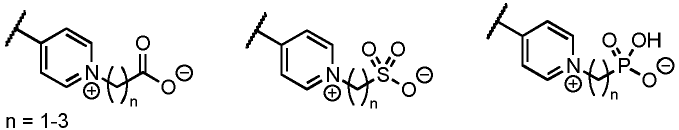

2. The lipid of claim 1, wherein Z-L has the structure of **Formula II** (linear linker structure):
X1-L_{b};
wherein L1' of **Formula IIIc** has 5 to 12, optionally 5 to 9, carbon atoms and has 0 to 3, optionally 0 to 2, cis or trans double bonds;
wherein G¹ of **Formula IIIc** is absent, CH₂ or CH=CHCH₂, and wherein the double bond is cis or trans;
wherein L1ʺʺ, if present, and S of **Formula IIIc** are independently selected from a linear or branched hydrocarbon with 0-5 cis or trans CH=CH and 2 to 18 carbon atoms;
and
wherein q of Formula IIIc is 1 to 9, optionally wherein q of Formula IIIc is 1 to 5;
optionally wherein each X1 of Formula IIIc is an ester oriented with an oxygen of the ester bonded to the L carbon backbone.

3. The lipid of claim 1, wherein (V)ₘ is (CH₂)ₘ, wherein m is 1 to 20;
Z-L has the structure of **Formula IIa** (branched linker structure): 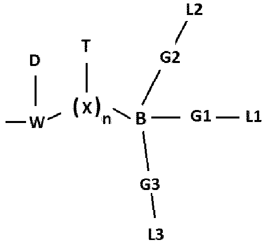
wherein W is an ether, ester or carbamate group and D is absent, and (X)ₙ is (CH₂)ₙ, wherein n is 1 to 10;
wherein G1 and G2 are present and are covalently bonded to the B via a (G)ᵤ, as B-(G)ᵤ-L1 or B-(G)ᵤ-L2, wherein (G)ᵤ is (CH₂)ᵤ;
wherein G3-L3 is present and is a hydrocarbon selected from CH₃ and CH₂CH₃; or wherein G3-L3 is CH₂X1L3 and L3 is a linear or branched hydrocarbon chain having 1 to 20 carbon atoms and 0 to 2 cis or trans double bonds or has the structure of **Formula IIIc.**

4. The lipid of claim 1, wherein Z-L has the structure of **Formula IIb:** 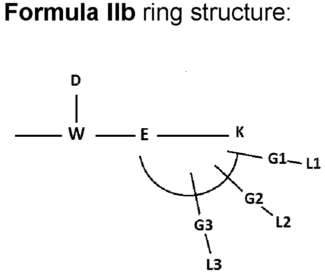 wherein:
the curved line represents a ring and E and K depict atoms that partially form the structure of the ring, which ring is a substituted or unsubstituted carbon ring having 3 to 6 ring atoms; or
the curved line represents a ring and E and K depict atoms that partially form the structure of the ring, which ring is a substituted or unsubstituted carbon ring having 3 to 6 ring atoms and the ring comprises 3 or 5 carbon atoms.

5. The lipid of claim 4, wherein at least L1 and L2 are present and are attached to the ring via respective G1 and G2 groups and wherein each G1 and G2 group is optionally covalently bonded to an atom of the ring via an intervening (G)ᵤ, wherein (G)ᵤ is (CH₂)ᵤ and u is 0 to 10 or 0 to 6.

6. The lipid of any one of claims 1 to 5, wherein the A moiety is -NR₂, wherein each R is independently methyl, ethyl, propyl, or butyl.

7. The lipid of any one of claims 1 to 6, wherein the lipid is capable of assembling into a lipid nanoparticle in combination with other lipids in aqueous solution; optionally wherein the other lipids are vesicle forming lipids and include phosphatidylcholine, phosphatidylglycerol, phosphatidylserine, phosphatidylethanolamine, phosphatidic acid, ceramides, sphingomyelin or a hydrophilic polymer-lipid conjugate.

8. The lipid of any one of claims 1 to 7, wherein:
the R1 or R2 of (V)ₘ is the cycloalkyl that is an optionally substituted mono-, bi-, or tricyclic carbon ring; or
the R1 or R2 of (V)ₘ are each independently: selected from the heteroatom ring having 4 to 12 ring atoms.

9. The lipid of any one of claims 1 to 7, wherein R1 and R2 of (V)ₘ are each hydrogen.

10. The lipid of claim 1, which has one of the following structures or a pharmaceutically acceptable salt thereof: 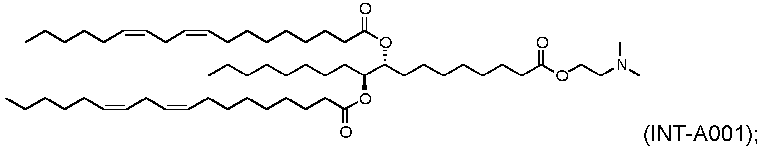 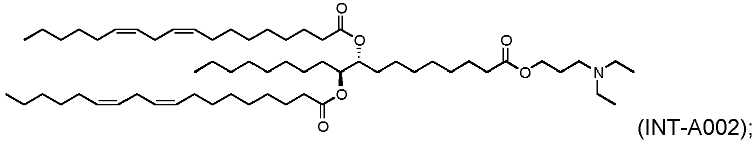 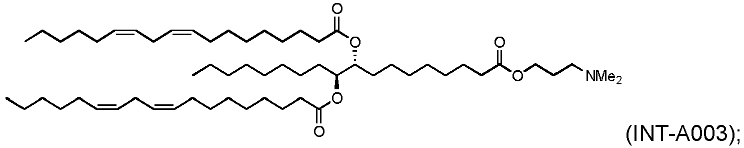 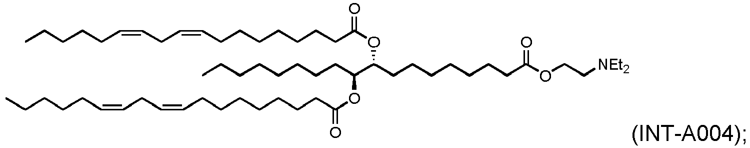 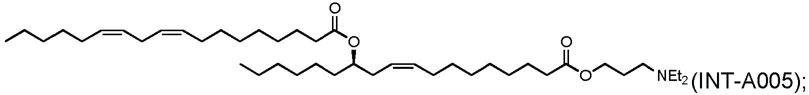 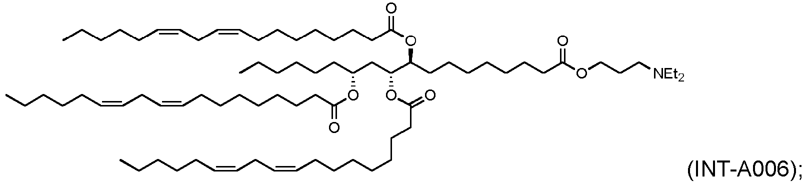 or

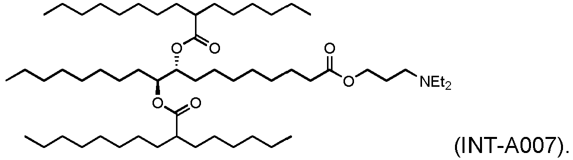

11. A drug delivery vehicle formulation comprising the lipid of any one of claims 1 to 10 incorporated in a lipid bilayer or monolayer thereof and comprising cargo molecule or compound that is a nucleic acid, protein or a peptide.

12. The drug delivery formulation of claim 11, wherein the nucleic acid is a small interfering RNA, a small activating RNA, a messenger RNA, a microRNA, an antisense oligonucleotide, a ribozyme, an aptamer, a plasmid, a circular DNA, a linear DNA, an antagomir, an anti-miRNA oligonucleotide or an miRNA mimic.

13. The drug delivery formulation of claim 11, wherein the cargo molecule or compound is a peptide.

14. The drug delivery vehicle formulation of claim 11, 12 or 13 comprising a lipid nanoparticle (LNP).

15. The drug delivery vehicle formulation of claim 13, wherein:
the polydispersity of the lipid nanoparticle is less than 0.15; and/or
the encapsulation efficiency of the lipid nanoparticle is: 70% to 100%, or 80% to 100%.

## Patentansprüche

1. Lipid, umfassend einen verzweigten Lipidanteil L, der die Struktur der **Formel I** aufweist, oder ein pharmazeutisch akzeptables Salz davon:
**Formel I:** A-(V)ₘ-Z-L,
wobei
A eine Kopfgruppe ist, die ionisierbar, permanent geladen oder zwitterionisch ist;
(V)ₘ ein optionales -(CR1R2)ₘ- ist und m 1 bis 10 oder 2 bis 6 ist, wobei R1 und R2 jeweils Folgendes unabhängig voneinander sind: Wasserstoff, optional substituiertes Alkyl, Alkenyl, Alkinyl, Aryl, Cycloalkyl, Cycloalkylalkyl oder ein Heterocyclus; und
Z-L eine Struktur der nachstehenden **Formel II, IIa** oder **IIb** aufweist,
und wobei L eine Kohlenwasserstoffstruktur ist und einen Anteil der nachstehenden Formel IIIc aufweist: 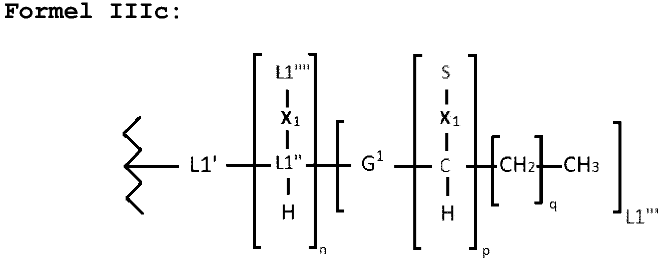
wobei eine Gerüstkohlenstoffkette von L durch L1' - L1"- G¹- CH-[CH₂]_{q} - CH₃ bezeichnet wird und wobei die Gesamtzahl von Kohlenstoffatomen in dem L-Kohlenstoffrückgrat 10 bis 30 ist;
L1' eine lineare Kohlenwasserstoffkette ist, die keine Heteroatome aufweist, und 5-12 Kohlenstoffatome und 0-3 cis- oder trans-Doppelbindungen aufweist;
L1" ein Kohlenstoffatom ist;
jedes X1 unabhängig voneinander aus einer Ether-, Ester- und Carbamatgruppe ausgewählt ist;
L1‴ ein Kohlenstoffrückgratabschnitt der Gerüstkohlenstoffkette L ist und durch G¹- CH-[CH₂]_{q}-CH₃ abgebildet ist und wobei G¹ eine Kohlenwasserstoffkette von 0-4 Kohlenstoffatomen ist, die optional eine cis- oder trans-Doppelbindung aufweist;
wobei n 0 bis 4 ist;
wobei p 1 bis 4 ist;
wobei n + p 1 bis 4 ist;
q 0 bis 20 ist;
wobei jedes S und L1"" eine Kohlenwasserstoffseitenkette ist and unabhängig voneinander Folgendes ist:
(a) eine lineare oder verzweigte beendende Kohlenwasserstoffkette mit 0 bis 5 cis oder trans C=C and 2 bis 30 Kohlenstoffatomen und konjugiert an eines von einem jeweiligen X1 an einem beliebigen Kohlenstoffatom in ihrer Kohlenwasserstoffkette davon; oder
(b) eine verzweigte Kohlenwasserstoffstruktur der **Formel IIIc,**
wobei jede eine der L1""- und S-Kohlenwasserstoffketten in dem Lipidanteil optional mit einem Heteroatom substituiert ist, mit der Maßgabe, dass nicht mehr als 2 Heteroatome in den Kohlenwasserstoffketten substituiert sind,
**Formel II** lineare Linker-Struktur: X1-L_{b},
wobei X1 optional ist und X1 aus einer Ether-, Ester- und Carbamatgruppe ausgewählt ist; und
L_{b} ein verzweigtes Lipid der **Formel IIIc** ist; 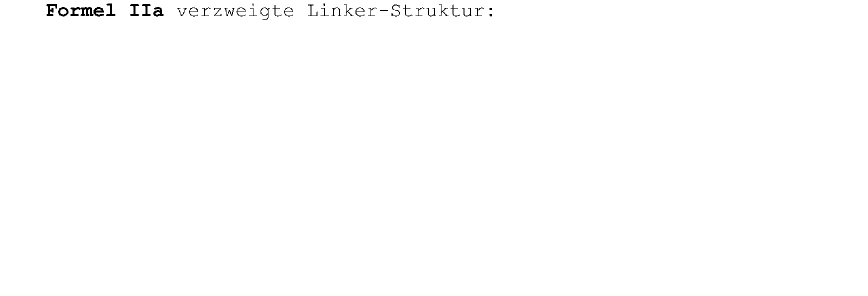 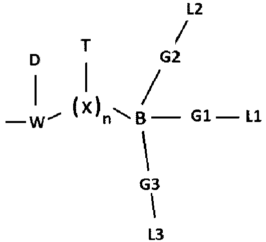
W optional ist;
W, falls vorhanden, eine X1-Verknüpfung, N-C(O), N-C(O)O, oder N-OC(O) ist;
wobei W optional mit D substituiert ist, das ein optional substituiertes Alkyl, Alkenyl, Alkinyl, Aryl, Cycloalkyl, Cycloalkylalkyl oder Heterocyclus ist;
jedes Vorkommen von (X)ₙ ein unabhängig voneinander ausgewähltes -(CR1R2)ₙ- ist; n von (X)ₙ 0 bis 10 ist; und T optional ist und ein Alkyl, Alkenyl, Alkinyl, Aryl, Cycloalkyl, Cycloalkylalkyl oder Heterocyclus ist und wobei T optional substituiert ist;
B ein Kohlenstoffatom ist, das über G1 bzw. G2 mit L1 und L2 verknüpft ist;
wobei G1 und G2 unabhängig voneinander aus einem X1 ausgewählt sind;
wobei jedes von G1 und G2 unabhängig voneinander optional über eine dazwischenliegende (G)ᵤ-Gruppe als B-(G)ᵤ-G1 bzw. B-(G)ᵤ-G2 kovalent an B gebunden ist;
wobei (G)ᵤ ein unabhängig voneinander ausgewähltes -(CR1R2)ᵤ-ist, wobei R1 und R2 jeweils unabhängig voneinander Folgendes sind: Wasserstoff, optional substituiertes Alkyl, Alkenyl, Alkinyl, Aryl, Cycloalkyl, Cycloalkylalkyl oder Heterocyclus und u 0 bis 16 ist;
wobei G3 optional ist und aus X1 ausgewählt ist und optional über eine dazwischenliegende (G)ᵤ-Gruppe als B-(G)ᵤ-G3 kovalent an das B gebunden ist;
L1 ein verzweigter Kohlenwasserstoff der **Formel IIIc** ist;
L2 eine Kohlenwasserstoffkette ist, die 1 bis 20 Kohlenstoffatome und 0 bis 2 cis- oder trans-Doppelbindungen aufweist, oder die Struktur der **Formel IIIc** aufweist;
L3, falls vorhanden, Wasserstoff, eine lineare oder verzweigte Kohlenwasserstoffkette, die 1 bis 20 Kohlenstoffatome und 0 bis 2 cis- oder trans-Doppelbindungen aufweist, ist oder die Struktur der **Formel IIIc** aufweist; 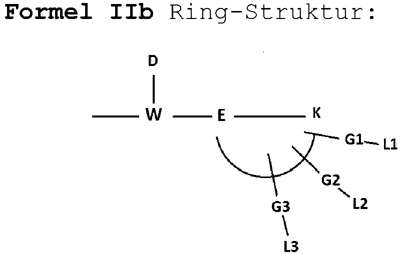
wobei die gekrümmte Linie einen Ring abbildet und E und K Atome darstellen, die teilweise die Struktur des Rings bilden, wobei der Ring ein substituierter oder unsubstituierter Ring ist, der 3 bis 8 Ringatome aufweist;
wobei mindestens eines von L1, L2 und L3 an ein einzelnes Atom in dem Ring gebunden sind, optional über ein jeweiliges G1, G2 und G3, wobei jedes von G1, G2 und G3 unabhängig voneinander optional über ein dazwischenliegendes (G)ᵤ kovalent an ein jeweiliges der L1, L2 und L3 gebunden ist, als G1-(G)ᵤ-L1, G2-(G)ᵤ-L2 bzw. G3-(G)ᵤ-L3;
wobei L1 und optional L2 und/oder L3 der **Formel IIb** die Struktur der **Formel IIIc** aufweisen; und
wobei der A-Anteil aus einem von Folgenden ausgewählt ist:
(i) ionisierbaren kationischen Anteilen, ausgewählt aus der Gruppe bestehend aus: 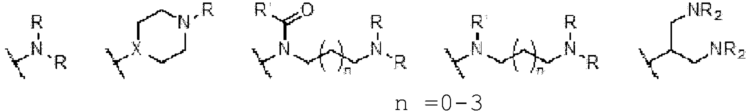 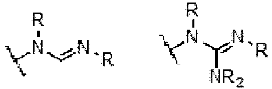
R = H, Me, Et, Pr, Bu
R' = C₅-C₁₇ Kohlenwasserstoff ohne *cis*/*trans-C=C*
X = N, CH
(ii) permanent geladenen Anteilen, ausgewählt aus der Gruppe bestehend aus: 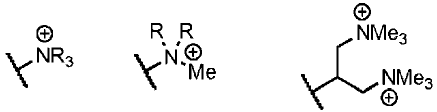 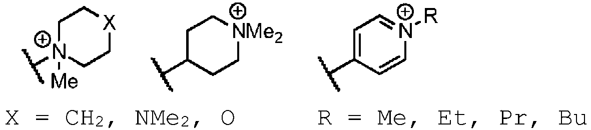
(iii) ionisierbaren anionischen Anteilen, ausgewählt aus der Gruppe bestehend aus: 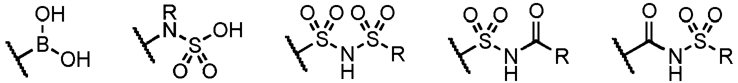 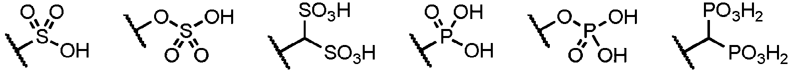 ;
oder
(iv) zwitterionischen Anteilen, ausgewählt aus der Gruppe bestehend aus: 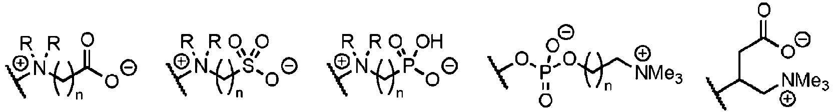
R = Me, Et, Pr, Bu
n = 1-3

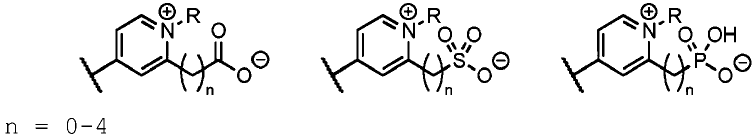

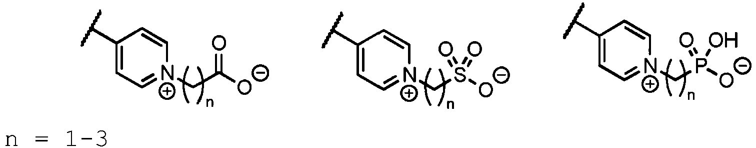

2. Lipid nach Anspruch 1, wobei Z-L die Struktur der **Formel II** (lineare Linker-Struktur) aufweist:
X1-L_{b};
wobei L1' der **Formel IIIc** 5 bis 12, optional 5 bis 9, Kohlenstoffatome aufweist und 0 bis 3, optional 0 bis 2, cis- oder trans-Doppelbindungen aufweist;
wobei G¹ der **Formel IIIc** nicht vorhanden ist, CH₂ oder CH=CHCH₂, und wobei die Doppelbindung cis oder trans ist;
wobei L1ʺʺ, falls vorhanden, und S der **Formel IIIc** unabhängig voneinander aus einem linearen oder verzweigten Kohlenwasserstoff mit 0-5 cis- oder trans-CH=CH und 2 bis 18 Kohlenstoffatomen ausgewählt sind;
und
wobei q der Formel IIIc 1 bis 9 ist, wobei optional q der Formel IIIc 1 bis 5 ist;
wobei optional jedes X1 der Formel IIIc ein Ester ist, der mit einem Sauerstoff des an das L-Kohlenstoffrückgrat gebundenen Esters ausgerichtet ist.

3. Lipid nach Anspruch 1, wobei (V)ₘ (CH₂)ₘ ist, wobei m 1 bis 20 ist;
Z-L die Struktur der **Formel IIa** (verzweigte Linker-Struktur) aufweist: 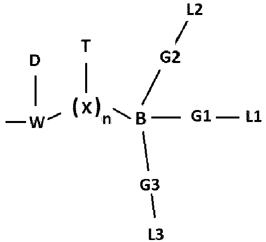
wobei W eine Ether-, Ester- oder Carbamatgruppe ist und D nicht vorhanden ist und (X)ₙ (CH₂)ₙ ist, wobei n 1 bis 10 ist;
wobei G1 und G2 vorhanden sind und über ein (G)ᵤ, als B-(G)ᵤ-L1 oder B-(G)ᵤ-L2 kovalent an das B gebunden sind, wobei (G)ᵤ (CH₂)ᵤ ist;
wobei G3-L3 vorhanden ist und ein Kohlenwasserstoff ist, der aus CH₃ und CH₂CH₃ ausgewählt ist; oder wobei G3-L3 CH₂X1L3 ist und L3 eine lineare oder verzweigte Kohlenwasserstoffkette ist, die 1 bis 20 Kohlenstoffatome und 0 bis 2 cis- oder trans-Doppelbindungen aufweist, oder die Struktur der **Formel IIIc** aufweist.

4. Lipid nach Anspruch 1, wobei Z-L die Struktur der **Formel IIb** aufweist: 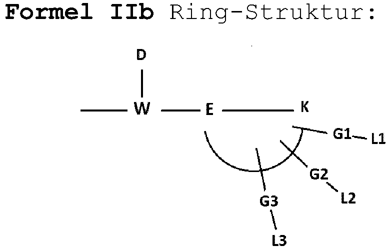 wobei:
die gekrümmte Linie einen Ring abbildet und E und K Atome darstellen, die teilweise die Struktur des Rings bilden, wobei der Ring ein substituierter oder unsubstituierter Kohlenstoffring ist, der 3 bis 6 Ringatome aufweist; oder die gekrümmte Linie einen Ring abbildet und E und K Atome abbilden, die teilweise die Struktur des Rings bilden, wobei der Ring ein substituierter oder unsubstituierter Kohlenstoffring ist, der 3 bis 6 Ringatome aufweist, und der Ring 3 oder 5 Kohlenstoffatome umfasst.

5. Lipid nach Anspruch 4, wobei mindestens L1 und L2 vorhanden sind und über jeweilige G1- und G2-Gruppen an dem Ring befestigt sind und wobei jede G1- und G2-Gruppe optional über ein dazwischenliegendes (G)ᵤ kovalent an ein Atom des Rings gebunden ist, wobei (G)ᵤ (CH₂)ᵤ ist und u 0 bis 10 oder 0 bis 6 ist.

6. Lipid nach einem der Ansprüche 1 bis 5, wobei der A-Anteil -NR₂ ist, wobei jedes R unabhängig voneinander Methyl, Ethyl, Propyl oder Butyl ist.

7. Lipid nach einem der Ansprüche 1 bis 6, wobei das Lipid in der Lage ist, in Kombination mit anderen Lipiden in wässriger Lösung zu einem Lipid-Nanopartikel zusammengesetzt zu werden; wobei die anderen Lipide optional vesikelbildende Lipide sind und Phosphatidylcholin, Phosphatidylglycerin, Phosphatidylserin, Phosphatidylethanolamin, Phosphatidsäure, Ceramide, Sphingomyelin oder ein hydrophiles Polymer-Lipid-Konjugat umfassen.

8. Lipid nach einem der Ansprüche 1 bis 7, wobei:
das R1 oder das R2 von (V)ₘ das Cycloalkyl ist, das ein optional substituierter mono-, bi- oder tricyclischer Kohlenstoffring ist; oder
das R1 oder das R2 von (V)ₘ jeweils Folgendes unabhängig voneinander sind: aus dem Heteroatomring, der 4 bis 12 Ringatome aufweist, ausgewählt.

9. Lipid nach einem der Ansprüche 1 bis 7, wobei R1 und R2 von (V)ₘ jeweils Wasserstoff sind.

10. Lipid nach Anspruch 1, das eine der folgenden Strukturen oder ein pharmazeutisch akzeptables Salz davon aufweist:

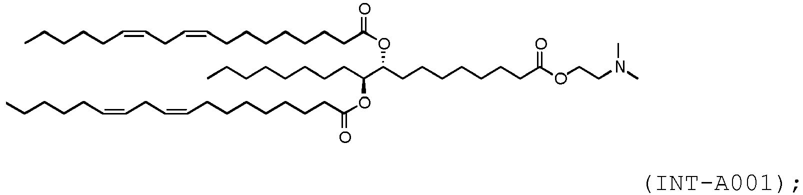

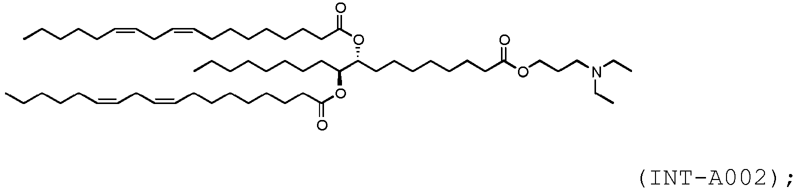

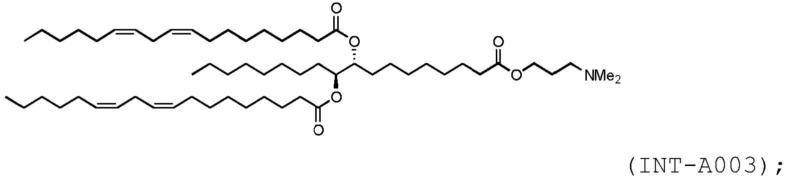

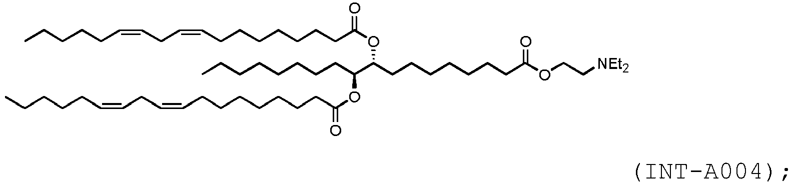

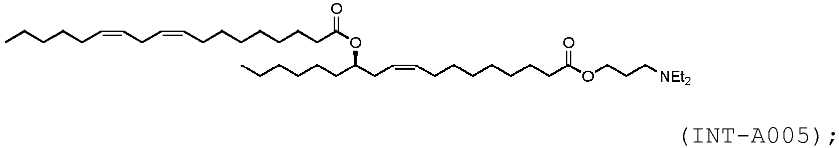

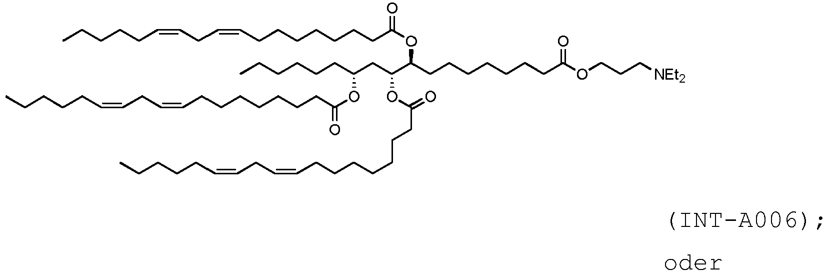

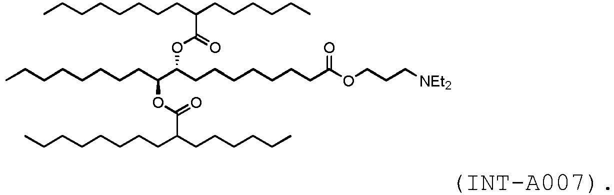

11. Arzneimittelabgabesystem-Formulierung, umfassend das Lipid nach einem der Ansprüche 1 bis 10, das in eine Lipid-Doppelschicht oder -Einzelschicht davon eingebaut ist, und umfassend ein Frachtmolekül oder eine Verbindung, die eine Nukleinsäure, ein Protein oder ein Peptid ist.

12. Arzneimittelabgabe-Formulierung nach Anspruch 11, wobei die Nukleinsäure eine kleine interferierende RNA, eine kleine aktivierende RNA, eine Boten-RNA, eine Mikro-RNA, ein Antisense-Oligonukleotid, ein Ribozym, ein Aptamer, ein Plasmid, eine zirkuläre DNA, eine lineare DNA, ein Antagomir, ein Anti-miRNA-Oligonukleotid oder ein miRNA Mimic ist.

13. Arzneimittelabgabe-Formulierung nach Anspruch 11, wobei das Frachtmolekül oder die Verbindung ein Peptid ist.

14. Arzneimittelabgabesystem-Formulierung nach Anspruch 11, 12 oder 13, umfassend ein Lipid-Nanopartikel (LNP).

15. Arzneimittelabgabesystem-Formulierung nach Anspruch 13, wobei:
die Polydispersität des Lipid-Nanopartikels weniger als 0,15 beträgt; und/oder
die Verkapselungseffizienz des Lipid-Nanopartikels Folgendes beträgt: 70 % bis 100 % oder 80 % bis 100 %.

## Revendications

1. Lipide comprenant un groupement lipide ramifié L ayant la structure de la **Formule I** ou un sel pharmaceutiquement acceptable de celui-ci :
**Formule I** : A-(V)ₘ-Z-L,
dans lequel
A est un groupe de tête qui est ionisable, chargé de façon permanente ou zwittérionique ;
(V)ₘ est un-(CR1R2)ₘ-facultatif, et m est 1 à 10 ou 2 à 6, dans lequel R1 et R2 sont chacun indépendamment : hydrogène, alkyle, alcényle, alcynyle, aryle, cycloalkyle, cycloalkylalkyleéventuellement substitué, ou un hétérocycle ; et
Z-L a une structure de **Formule II, IIa** ou **IIb** ci-dessous, et dans lequel L est une structure hydrocarbonée et a un groupement de Formule IIIc ci-dessous : 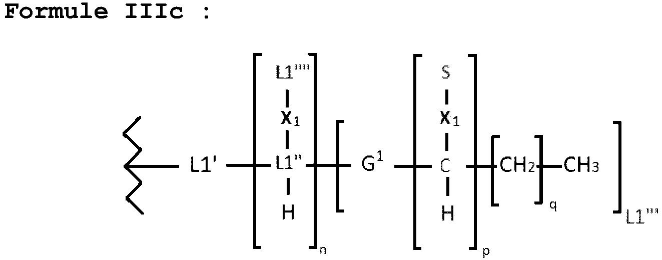
dans lequel une chaîne carbonée d'échafaudage de L est désignée par L1'-L1"-G¹-CH-[CH₂]_{q}-CH₃, et dans lequel le nombre total d'atomes de carbone dans la chaîne principale carbonée L est de 10 à 30 ;
L1' est une chaîne hydrocarbonée linéaire n'ayant aucun hétéroatome et comporte 5-12 atomes de carbone et 0-3 doubles liaisons cis ou trans ;
L1" est un atome de carbone ;
chaque X1 est indépendamment choisi parmi un groupe éther, ester et carbamate ;
L1‴ est une partie de chaîne principale carbonée de la chaîne carbonée d'échafaudage L et est représenté par G¹-CH-[CH₂]_{q}-CH₃ et dans lequel G¹ est une chaîne hydrocarbonée de 0-4 atomes de carbone, ayant éventuellement une double liaison cis ou trans ;
dans lequel n est 0 à 4 ;
dans lequel p est 1 à 4 ;
dans lequel n + p est 1 à 4 ;
q est 0 à 20 ;
dans lequel chaque S et L1"" est une chaîne latérale hydrocarbonée et est indépendamment :
(a) une chaîne hydrocarbonée terminale linéaire ou ramifiée avec 0 à 5 C=C cis ou trans et 2 à 30 atomes de carbone et conjuguée à l'un d'un X1 respectif au niveau de n'importe quel atome de carbone dans sa chaîne hydrocarbonée de celle-ci ; ou
(b) une structure hydrocarbonée ramifiée de **Formule IIIc,**
dans lequel chacune des chaînes hydrocarbonées L1"" et S dans le groupement lipide est éventuellement substituée par un hétéroatome, à la condition que pas plus de 2 hétéroatomes soient substitués dans les chaînes hydrocarbonées,
**Formule II** structure de lieur linéaire : X1-L_{b},
dans lequel X1 est facultatif et X1 est choisi parmi un groupe éther, ester et carbamate ; et
L_{b} est un lipide ramifié de **Formule IIIc** ; 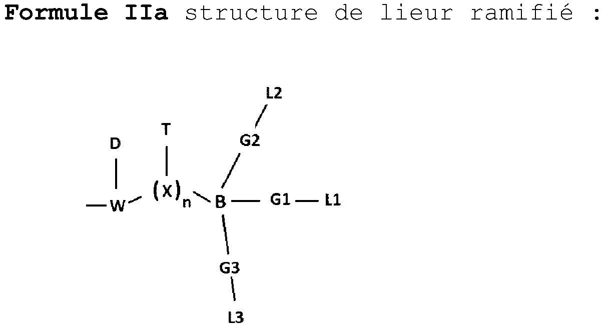
W est facultatif ;
W, si présent, est une liaison X1, N-C(O), N-C(O)O, ou N-OC(O) ; dans lequel W est éventuellement substitué par D, qui est un alkyle, alcényle, alcynyle, aryle, cycloalkyle, cycloalkylalkyle ou hétérocycle éventuellement substitué ;
chaque occurrence de (X)ₙ est un-(CR1R2)ₙ choisi indépendamment ;
n de (X)ₙ est 0 à 10 ; et T est facultatif et est un alkyle, alcényle, alcynyle, aryle, cycloalkyle, cycloalkylalkyle ou hétérocycle et dans lequel T est éventuellement substitué ;
B est un atome de carbone lié à L1 et L2 via G1 et G2 respectifs ;
dans lequel G1 et G2 sont indépendamment choisis parmi un X1 ;
dans lequel chacun de G1 et G2 est indépendamment éventuellement lié de façon covalente à B via un groupe (G)ᵤ intermédiaire sous la forme B-(G)ᵤ-G1 ou B-(G)ᵤ-G2, respectivement ;
dans lequel (G)ᵤ est un-(CR1R2)ᵤ-choisi indépendamment dans lequel R1 et R2 sont chacun indépendamment : hydrogène, alkyle, alcényle, alcynyle, aryle, cycloalkyle, cycloalkylalkyle ou hétérocycle éventuellement substitué et u est 0 à 16 ;
dans lequel G3 est facultatif et est choisi parmi X1 et éventuellement lié de façon covalente au B via un groupe (G)ᵤ intermédiaire sous la forme B-(G)ᵤ-G3 ;
L1 est un hydrocarbure ramifié de **Formule IIIc** ;
L2 est une chaîne hydrocarbonée comportant 1 à 20 atomes de carbone et 0 à 2 doubles liaisons cis ou trans ou a la structure de la **Formule IIIc** ;
L3 si présent est hydrogène, une chaîne hydrocarbonée linéaire ou ramifiée comportant 1 à 20 atomes de carbone et 0 à 2 doubles liaisons cis ou trans ou a la structure de la **Formule IIIc** ; 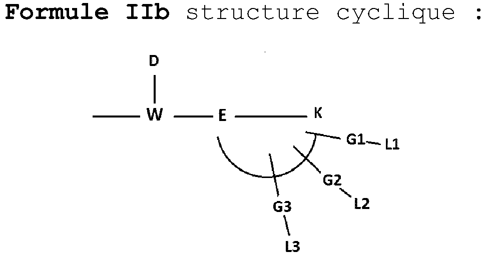
dans lequel la ligne courbe représente un cycle et E et K représentent des atomes qui forment partiellement la structure du cycle, lequel cycle est un cycle substitué ou non substitué comportant 3 à 8 atomes de cycle ;
dans lequel au moins l'un de L1, L2 et L3 est lié à un atome unique dans le cycle, éventuellement via un G1, G2 et G3 respectif, dans lequel chacun de G1, G2 et G3 est indépendamment éventuellement lié de façon covalente à l'un respectif des L1, L2 et L3 via un (G)ᵤ intermédiaire, sous la forme G1-(G)ᵤ-L1, G2-(G)ᵤ-L2 ou G3-(G)ᵤ-L3, respectivement ;
dans lequel L1 et éventuellement L2 et/ou L3 de **Formule IIb** ont la structure de **Formule IIIc** ; et
dans lequel le groupement A est choisi parmi l'un de :
(i) des groupements cationiques ionisables choisis dans le groupe constitué par : 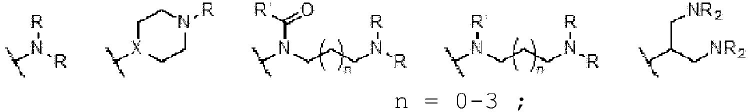 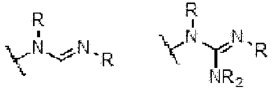
R = H, Me, Et, Pr, Bu ;
R' = hydrocarbure en C₅-C₁₇ sans C=C cis/trans ;
X = N, CH ;
(ii) des groupements chargés de façon permanente choisis dans le groupe constitué par : 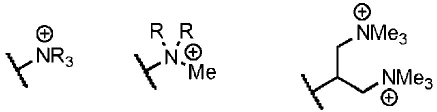 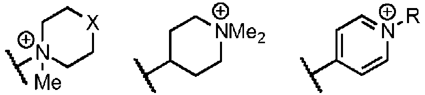 X = CH₂, NMe₂, O R = Me, Et, Pr, Bu ;
(iii) des groupements anioniques ionisables choisis dans le groupe constitué par : 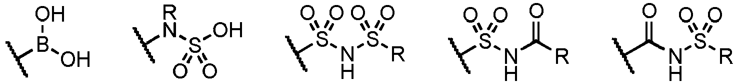 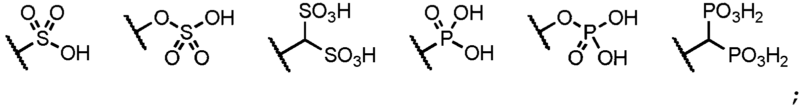 ou
(iv) des groupements zwittérioniques choisis dans le groupe constitué par :

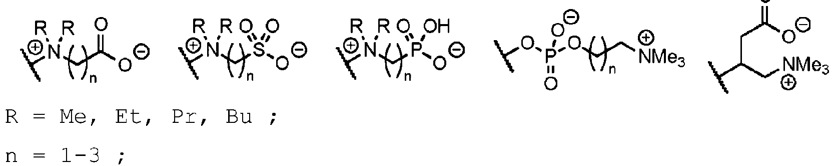

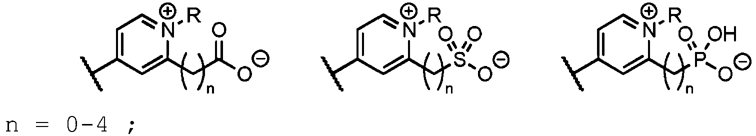

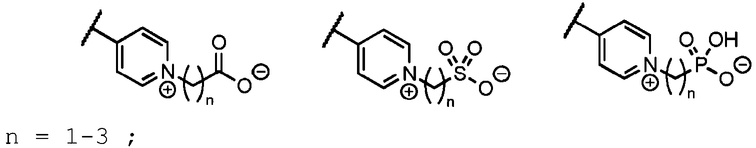

2. Lipide selon la revendication 1, dans lequel Z-L a la structure de **Formule II** (structure de lieur linéaire) :
X1-L_{b} ;
dans lequel L1' de **Formule IIIc** comporte 5 à 12, éventuellement 5 à 9, atomes de carbone et comporte 0 à 3, éventuellement 0 à 2, doubles liaisons cis ou trans ;
dans lequel G¹ de **Formule IIIc** est absent, CH₂ ou CH=CHCH₂, et
dans lequel la double liaison est cis ou trans ;
dans lequel L1ʺʺ, si présent, et S de **Formule IIIc** sont indépendamment choisis parmi un hydrocarbure linéaire ou ramifié avec 0-5 CH=CH cis ou trans et 2 à 18 atomes de carbone ;
et
dans lequel q de Formule IIIc est 1 à 9, éventuellement dans lequel q de Formule IIIc est 1 à 5 ;
éventuellement dans lequel chaque X1 de Formule IIIc est un ester orienté avec un oxygène de l'ester lié à la chaîne principale carbonée L.

3. Lipide selon la revendication 1, dans lequel (V)ₘ est (CH₂)ₘ, dans lequel m est 1 à 20 ;
Z-L a la structure de **Formule IIa** (structure de lieur ramifié) : 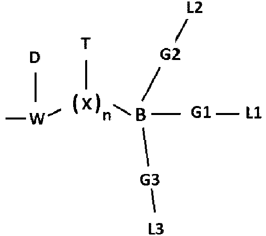
dans lequel W est un groupe éther, ester ou carbamate et D est absent, et (X)ₙ est (CH₂)ₙ, dans lequel n est 1 à 10 ;
dans lequel G1 et G2 sont présents et sont liés de façon covalente au B via un (G)ᵤ, sous la forme B-(G)ᵤ-L1 ou B-(G)ᵤ-L2, dans lequel (G)ᵤ est (CH₂)ᵤ ;
dans lequel G3-L3 est présent et est un hydrocarbure choisi parmi CH₃ et CH₂CH₃ ; ou dans lequel G3-L3 est CH₂X1L3 et L3 est une chaîne hydrocarbonée linéaire ou ramifiée comportant 1 à 20 atomes de carbone et 0 à 2 doubles liaisons cis ou trans ou a la structure de la **Formule IIIc.**

4. Lipide selon la revendication 1, dans lequel Z-L a la structure de **Formule IIb** : 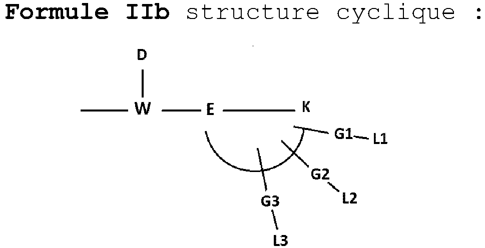 dans lequel :
la ligne courbe représente un cycle et E et K représentent des atomes qui forment partiellement la structure du cycle, lequel cycle est un cycle carboné substitué ou non substitué comportant 3 à 6 atomes de cycle ; ou la ligne courbe représente un cycle et E et K représentent des atomes qui forment partiellement la structure du cycle, lequel cycle est un cycle carboné substitué ou non substitué comportant 3 à 6 atomes de cycle et le cycle comprend 3 ou 5 atomes de carbone.

5. Lipide selon la revendication 4, dans lequel au moins L1 et L2 sont présents et sont attachés au cycle via des groupes G1 et G2 respectifs et dans lequel chaque groupe G1 et G2 est éventuellement lié de façon covalente à un atome du cycle via un (G)ᵤ intermédiaire, dans lequel (G)ᵤ est (CH₂)ᵤ et u est 0 à 10 ou 0 à 6.

6. Lipide selon l'une quelconque des revendications 1 à 5, dans lequel le groupement A est-NR₂, dans lequel chaque R est indépendamment méthyle, éthyle, propyle ou butyle.

7. Lipide selon l'une quelconque des revendications 1 à 6, dans lequel le lipide est capable de s'assembler en une nanoparticule lipidique en combinaison avec d'autres lipides en solution aqueuse ; éventuellement dans lequel les autres lipides sont des lipides formant des vésicules et incluent la phosphatidylcholine, le phosphatidylglycérol, la phosphatidylsérine, la phosphatidyléthanolamine, l'acide phosphatidique, des céramides, la sphingomyéline ou un conjugué polymère hydrophile-lipide.

8. Lipide selon l'une quelconque des revendications 1 à 7, dans lequel :
le R1 ou R2 de (V)ₘ est le cycloalkyle qui est un cycle carboné mono-, bi-ou tricyclique éventuellement substitué ; ou
les R1 ou R2 de (V)ₘ sont chacun indépendamment : choisis parmi le cycle à hétéroatome comportant 4 à 12 atomes de cycle.

9. Lipide selon l'une quelconque des revendications 1 à 7, dans lequel R1 et R2 de (V)ₘ sont chacun hydrogène.

10. Lipide selon la revendication 1, qui a l'une des structures suivantes ou un sel pharmaceutiquement acceptable de celui-ci :

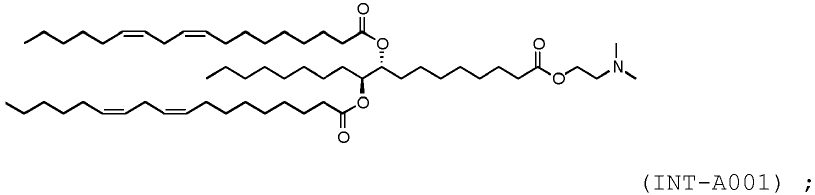

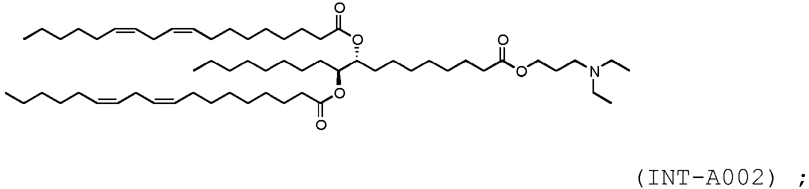

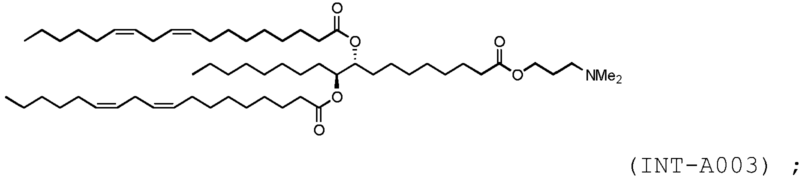

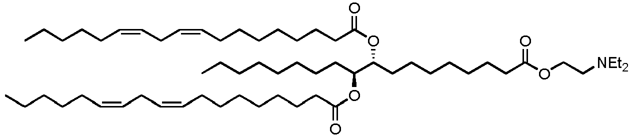

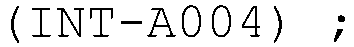

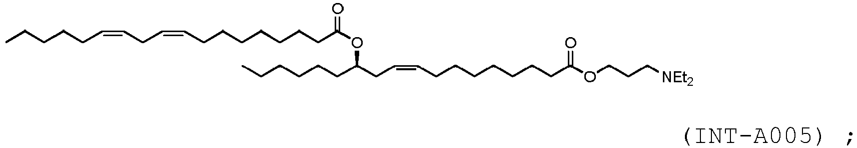

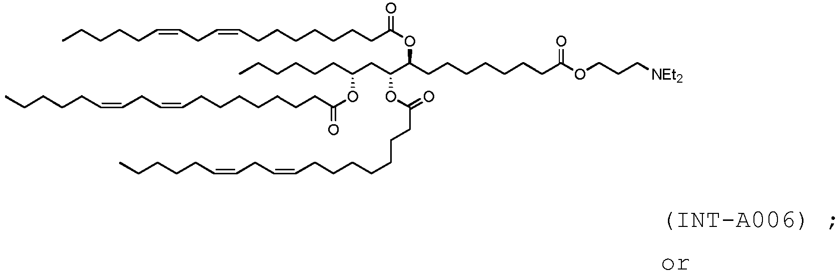

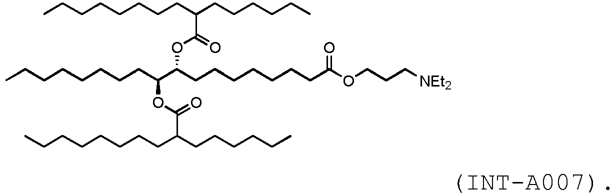

11. Formulation de véhicule d'administration de médicament comprenant le lipide selon l'une quelconque des revendications 1 à 10 incorporé dans une bicouche ou monocouche lipidique de celle-ci et comprenant une molécule ou un composé de cargaison qui est un acide nucléique, une protéine ou un peptide.

12. Formulation de véhicule d'administration de médicament selon la revendication 11, dans laquelle l'acide nucléique est un ARN interférent court, un ARN activateur court, un ARN messager, un micro-ARN, un oligonucléotideantisens, un ribozyme, un aptamère, un plasmide, un ADN circulaire, un ADN linéaire, un antagomire, un oligonucléotide anti-miARN ou un mimétique de miARN.

13. Formulation de véhicule d'administration de médicament selon la revendication 11, dans laquelle la molécule ou le composé de cargaison est un peptide.

14. Formulation de véhicule d'administration de médicament selon la revendication 11, 12 ou 13 comprenant une nanoparticule lipidique.

15. Formulation de véhicule d'administration de médicamentselon la revendication 13, dans laquelle :
la polydispersité de la nanoparticule lipidique est inférieure à 0,15 ; et/ou
l'efficacité d'encapsulation de la nanoparticule lipidique est : 70 % à 100 %, ou 80 % à 100 %.
